# EUROPEAN PATENT SPECIFICATION

(11) **EP 3 344 655 B1**
(45) Date of publication and mention of the grant of the patent: **26.07.2023**
(21) Application number: 16763663.8
(22) Date of filing: 31.08.2016
(51) Int. Cl.: C07K 16/28, A61K 39/395

(54) **USE OF ANTI-CD40 ANTIBODIES FOR TREATMENT OF LUPUS NEPHRITIS**
VERWENDUNG VON ANTI-CD40-ANTIKÖRPERN ZUR BEHANDLUNG VON LUPUS NEPHRITIS
UTILISATION D'ANTICORPS ANTI-CD40 POUR LE TRAITEMENT DE LA NÉPHRITE LUPIQUE

(30) Priority: 01.09.2015 US 201562212810 P; 19.11.2015 US 201562257336 P; 27.01.2016 US 201662287587 P
(43) Date of publication of application: 11.07.2018
(73) Proprietor: Boehringer Ingelheim International GmbH, 55216 Ingelheim am Rhein (DE)
(72) Inventor: BRODEUR, Scott Ronald, Ridgefield, Connecticut 06877-0368 (US); FREEMAN, Thomas B., San Bruno, California 94066 (US); NABOZNY, Gerald Henry, Ridgefield, Connecticut 06877-0368 (US); RAMANUJAM, Meera, Ridgefield, Connecticut 06877-0368 (US); SCHOLL, Paul, Ridgefield, Connecticut 06877-0368 (US); STEFFGEN, Juergen, 55216 Ingelheim am Rhein (DE)
(74) Representative: Wallinger Ricker Schlotter Tostmann
(86) International application number: PCT/US2016/049558
(87) International publication number: WO 2017/040566

(56) References cited:
- WO-A2-2006/073443
- WO-A2-2006/128103
- WO-A2-2007/124299
- WO-A2-2012/149356
- US-A1- 2003 059 427
- US-A1- 2011 243 932
- ÈLIA RIPOLL ET AL: "CD40 Gene Silencing Reduces the Progression of Experimental Lupus Nephritis Modulating Local Milieu and Systemic Mechanisms", PLOS ONE, vol. 8, no. 6, 14 June 2013 (2013-06-14), page e65068, XP055166673, DOI: 10.1371/journal.pone.0065068
- MOHAN C ET AL: "INTERACTION BETWEEN CD40 AND ITS LIGAND GP39 IN THE DEVELOPMENT OF MURINE LUPUS NEPHRITIS", THE JOURNAL OF IMMUNOLOGY, THE AMERICAN ASSOCIATION OF IMMUNOLOGISTS, US, vol. 154, no. 3, 1 January 1995 (1995-01-01), pages 1470-1480, XP002062342, ISSN: 0022-1767
- ALMAGRO JUAN C ET AL: "Humanization of antibodies", FRONTIERS IN BIOSCIENCE,, vol. 13, 1 January 2008 (2008-01-01), pages 1619-1633, XP002614477, ISSN: 1093-4715

## Description

### FIELD OF THE INVENTION

This invention generally relates to the use of humanized anti-CD40 antibodies for the treatment and/or prevention of lupus nephritis.

### BACKGROUND OF THE INVENTION

CD40 is a 48kDa type I integral membrane glycoprotein and a member of the tumor necrosis factor (TNF) receptor superfamily. CD40 is expressed on a variety of cell types including normal and neoplastic B cells, interdigitating cells, carcinomas, epithelial cells (e.g. keratinocytes), fibroblasts (e.g. synoviocytes) and platelets. It is also present on monocytes, macrophages, some endothelial cells, and follicular dendritic cells. CD40 is expressed early in B cell ontogeny, appearing on B cell precursors subsequent to the appearance of CD10 and CD19, but prior to expression of CD21, CD23, CD24, and appearance of surface immunoglobulin M (slgM) (Uckun et al., 1990, Blood 15:2449). CD40 has also been detected on tonsil and bone marrow-derived plasma cells (Pellat-Decounynck et al., 1994, Blood 84:2597).

The ligand of CD40 is CD40L (also referred to as CD154, gp39, and TRAP), a TNF superfamily member. CD40L is a transmembrane protein expressed predominantly on activated CD4⁺ T cells and a small subset of CD8+ T cells (Reviewed by (Van Kooten C. and Banchereau, 2000).

The interaction of CD40 with CD40L induces both humoral and cell-mediated immune responses. CD40 regulates this ligand-receptor pair to activate B cells and other antigen-presenting cells (APC) including dendritic cells (DCs) (Reviewed by (Toubi and Shoenfeld, 2004); (Kiener, et al., 1995). The function of CD40 on B cells has been studied extensively. Activation of CD40 on B cells induces proliferation, differentiation into antibody secreting cells and isotype switching in germinal centers of secondary lymphoid organs. *In vitro* studies have shown direct effects of CD40 activation on cytokine production (IL-6, IL-10, TNF-α, LT-α), expression of adhesion molecules and costimulatory receptors (ICAM, CD23, CD80 and CD86), and increased expression of MHC class I, MHC class II, and TAP transporter by B lymphocytes (Liu, et al., 1996). For most of these processes, CD40 acts in concert with either cytokines or other receptor-ligand interactions.

CD40 signaling on monocytes and DCs results in enhanced survival as well as secretion of cytokines (IL-1, IL-6, IL-8, IL-10, IL-12, TNF-α and MIP-1α). CD40 ligation on these APCs also leads to the up-regulation of costimulatory molecules such as (ICAM-1, LFA-3, CD80, and CD86). Activation of CD40 receptors is one of the critical signals that allow the full maturation of DC into efficient APCs driving T cell activation (Banchereau and Steinman, 1998) (Van Kooten C. and Banchereau, 2000).

Recent studies in mouse models showed that CD40 signaling on dendritic cells also plays an important role in the generation of TH17 cells which are considered as mediators of autoimmunity in diseases such as arthritis and multiple sclerosis (lezzi, et al., 2009) (Perona-Wright, et al., 2009).

The availability of CD40 and CD40L knock-out mice as well as agonistic and antagonistic anti-mouse antibodies offered the possibility to study the role of CD40-CD40L interactions in several disease models. Administration of blocking anti-CD40L has been demonstrated to be beneficial in several models of autoimmunity including spontaneous diseases like lupus nephritis in SNF1 mice or diabetes in NOD mice or in experimentally induced forms of disease like collagen-induced arthritis (CIA) or experimental autoimmune encephalomyelitis (EAE) (Toubi and Shoenfeld, 2004). CIA in mice was inhibited by an anti-CD40L mAb which blocked the development of joint inflammation, serum antibody titers to collagen, the infiltration of inflammatory cells into the subsynovial tissue in addition to the erosion of cartilage and bone (Durie, et al., 1993). Both for lupus nephritis and EAE, it was demonstrated that anti-CD40L could also alleviate ongoing disease, confirming the role of CD40-CD40L in the effector phase of the disease (Kalled, et al., 1998); (Howard, et al., 1999). Antibodies interfring with CD40-CD40L interaction are further disclosed in WO 2007/124299 A2, WO 2012, 149356 A2, US 2003/059427 A1, WO 2006/073443 A2, WO 2006/128103 and Mohan et al., (The American Association of Immunologists, US, 1995, 154 (3): 1470-1480).

Thus, preclinical studies that provide evidence for the crucial role of the CD40-CD40L dyad in driving an efficient T cell-dependent immune response. Blocking of CD40 signaling is therefore recognized as a suitable and needed therapeutic strategy to suppress a pathogenic autoimmune response in diseases such as lupus nephritis.

The International Society of Nephrology (ISN)/Renal Pathology Society provided a classification for lupus nephritis based in part on the degree of renal impairment. (See J.J. Weening et al., "The classification of glomerulonephritis in systemic lupus erythematosus revisited," J. Am. Soc. Nephrol 15: 241-250, 2004.)

Renal biopsies from SLE patients with Lupus Nephritis show a spectrum of vascular, glomerular, and tubulointerstitial lesions. The glomerular injury is determined by immune-complex localization on mesangial, endothelial and epithelial cells. Depending on these changes Lupus Nephritis can be divided into 6 classes ranging from relatively mild impairment showing only immuncomplex deposition in the mesangial matrix (class I) or mesangial hyper cellularity (class II) to global sclerosis in >90% of the glomerula (class VI). Patients with class I or II lupus nephritis show only asymptomatic proteinuria and no decline in GFR. Endocapillary changes are classified as either focal proliferative (class III) or diffuse proliferative (>50% of glomerula affected, class IV). Clinical findings in these patients are hematuria, symptomatic proteinuria and loss of GFR. Pure epithelial damage (Class V) results in immunocomplex deposition along the glomerular basal membrane without inflammatory lesions. These patients are mainly suffering from nephrotic proteinuria.

There is no need for immunosuppressive treatment of Class I and II and no effect of treatment after irreversible damage in class VI, whereas lupus nephritis III-V are accessible to immunosuppressive therapy. About 25% of lupus nephritis patients are class III, 40% class IV and 10% class V. About 1/6 of class III and IV will also have class 5 (overlap).

Immunosuppressive treatment should be guided by renal biopsy, and aiming for complete renal response (proteinuria <0.5 g/24 h with normal or near-normal renal function). Besides glucocorticoids, there is no approved therapy of lupus nephritis, however, cyclophosphamide or mycophenolate (MMF) each in combination with glucocorticoids have been shown to induce improve clinical symptoms of active lupus nephritis and to reduce the risk of progression to ESRD. About 20-30% of patients treated with MMF or cyclophosphamide show a complete renal response (proteinuria <0.5 g/d, no active sediment, no worsening of GFR) and another about 20% show at least a partial response (reduction of proteinuria by ≥ 50%).

Therefore the combination of glucocorticosteroids with either MMF or cyclophosphamide is looked upon as current standard of care for lupus nephritis which is also reflected by the recommendations of the Joint European League Against Rheumatism and European Renal Association-European Dialysis and Transplant Association (EULAR/ERA-EDTA). These recommendations include also hydroxychloroquine for all patients with lupus nephritis to reduce the number of flares and the use of ACE-inhibitors or ARBs.

However, all treatments available for lupus nephritis may be associated with significant toxicity (for example, infertility, infection, malignancy). Furthermore the complete response rates remain low and within the responders there is a high rate of relapses justifying long-term maintenance therapy. Recently conducted Phase III trials in lupus nephritis (e.g. rituximab, abatacept) have failed to meet their primary endpoints. CD40 gene silencing has been reported to modulate SLE progression leading to Lupus nephritis in NZB/W F1 mice without showing any local effect on renal or kidney cells or extracellular interference with CD40 interaction. Taken together, there is a high unmet need for new therapies in lupus nephritis. This need could be addressed by the humanized anti-CD40 antibodies described herein and in US20110243932 that specifically bind CD40 and which show the antigen binding specificity, affinity, and pharmacokinetic and pharmacodynamic properties that allow use thereof in therapeutic intervention of lupus nephritis.

### BRIEF SUMMARY OF THE INVENTION

The present invention provides a humanized monoclonal antibody for use in a method to treat and/or prevent lupus nephritis ("the method of the invention"). In all aspects and embodiments, said humanized monoclonal antibody is as specified in the claims. Any references in the description to methods of treatment refer to the compounds, pharmaceutical compositions and medicaments of the present invention for use in a method for treatment of the human (or animal) body by therapy (or for diagnosis).

Specifically, the present invention relates to a humanized antibody that specifically binds human CD40 for use in treating or preventing lupus nephritis, wherein said antibody comprises a heavy chain variable region of SEQ ID NO: 53 and a light chein variable region of SEQ ID NO: 52. In certain embodiments, the antibody is an IgG1 antibody and optionally the antibody has a Leu234Ala and Leu235Ala mutation in the Fc region and comprises a kappa light chain.

In certain embodiments, the antibody used in the method of treating or preventing lupus nephritis is a humanized antibody having the heavy chain variable region amino acid sequence of SEQ ID NO:35 or SEQ ID NO: 53, and the antibody used in the method of the invention is a humanized antibody that comprises a light chain variable domain amino acid sequence of SEQ ID NO:31 or SEQ ID NO:52.

Also contemplated is the anti-CD40 antibody for use in treating and/or preventing lupus nephritis, wherein the anti-CD40 antibody comprises a humanized antibody having a heavy chain variable domain and a light chain variable region comprising the amino acid sequences of SEQ ID NO:35 and SEQ ID NO:31.

In another embodiment, the humanized antibody used in the method of of treating or preventing lupus nephritis comprises a heavy chain variable domain and a light chain variable region comprising the amino acid sequences of SEQ ID NO:35 and SEQ ID NO:31, respectively.

Another embodiment, the antibody relates to the treatment and/or prevention of lupus nephritis using an isolated antibody that specifically binds to human CD40, wherein the isolated antibody comprises a humanized heavy chain variable domain comprising a framework region having an amino acid sequence of the framework region of the human variable domain heavy chain amino acid sequence of SEQ ID NO:35, and comprising a light chain amino acid sequence of SEQ ID NO:31.

Also described herein is the use of the humanized monoclonal antibody described herein for the preparation of a medicament to treat and/or prevent lupus nephritis ("the method of the invention").

In other words the present description relates to the use of the humanized monoclonal antibody described herein in the manufacture of a pharmaceutical composition for the treatment and/or prevention of lupus nephritis.

Expressed again differently the present invention relates to the humanized monoclonal antibody described herein for use in the treatment and/or prevention of lupus nephritis. The scope of the present invention is defined by the claims and any information that does not fall within the claims is provided for information only.

### BRIEF DESCRIPTION OF THE SEVERAL VIEWS OF THE DRAWINGS

Fig. 1 shows the effect of CD40 ligation on human endothelial cells (HUVEC) cells in a media comprising 100ng/ml of sCD40L and either Antibody B (●) or isotype control (lgG1 human antibody) (▲).
Fig. 2 shows the effect of CD40 ligation on human proximal tubular epithelial cells (PTEC) from three different donors (D1, D2, D3) in a media containing 50ng/ml of sCD40 Antibody B.

### DETAILED DESCRIPTION OF THE INVENTION

CD40 mediated signalling is now recognized as being involved in a variety of target disorders. Despite the availability of a variety of preclinical data showing that intervention in these disorders would be therapeutically beneficial, there remains a need for antagonistic anti-CD40 antibodies that can be used in the treatment of autoimmune diseases such as lupus nephritis.

The terms "CD40" and "CD40 surface antigen" refer to an approximately 48 kD glycoprotein expressed on the surface of normal and neoplastic B cells, which acts as a receptor for signals involved in cellular proliferation and differentiation (Ledbetter et al., 1987, J. Immunol. 138:788-785). A cDNA molecule encoding CD40 has been isolated from a library prepared from the Burkitt lymphoma cell line Raji (Stamenkovic et al., 1989, EMBO J. 8:1403).

As used herein, a cell that endogenously expresses CD40 is any cell characterized by the surface expression of CD40, including, but not limited to, normal and neoplastic B cells, interdigitating cells, basal epithelial cells, carcinoma cells, macrophages, endothelial cells, follicular dendritic cells, tonsil cells, and bone marrow-derived plasma cells. In some embodiments, the CD40 molecule is a human CD40 molecule.

The antibodies of the invention specifically bind to human recombinant and native CD40. A humanized monoclonal antibody wherein said antibody specifically binds to human CD40 having an antagonistic activity IC50 of less than 1nM and has no agonism up to 100 µg/ml in B cell proliferation and wherein said antibody is further characterized in that the antibody has an in vivo half-life in non-human primates that is at least 10 days.

The generalized structure of antibodies or immunoglobulin is well known to those of skill in the art, these molecules are heterotetrameric glycoproteins, typically of about 150,000 daltons, composed of two identical light (L) chains and two identical heavy (H) chains. Each light chain is covalently linked to a heavy chain by one disulfide bond to form a heterodimer, and the heterotrameric molecule is formed through a covalent disulfide linkage between the two identical heavy chains of the heterodimers. Although the light and heavy chains are linked together by one disulfide bond, the number of disulfide linkages between the two heavy chains varies by immunoglobulin isotype. Each heavy and light chain also has regularly spaced intrachain disulfide bridges. Each heavy chain has at the amino-terminus a variable domain (V_{H}), followed by three or four constant domains (C_{H1}, C_{H2}, C_{H3}, and C_{H4}), as well as a hinge region between C_{H1} and C_{H2}. Each light chain has two domains, an amino-terminal variable domain (V_{L}) and a carboxy-terminal constant domain (C_{L}). The V_{L} domain associates non-covalently with the V_{H} domain, whereas the C_{L} domain is commonly covalently linked to the C_{H1} domain via a disulfide bond. Particular amino acid residues are believed to form an interface between the light and heavy chain variable domains (Chothia et al., 1985, J. Mol. Biol. 186:651-663.)

Certain domains within the variable domains differ extensively between different antibodies i.e., are "hypervariable." These hypervariable domains contain residues that are directly involved in the binding and specificity of each particular antibody for its specific antigenic determinant. Hypervariability, both in the light chain and the heavy chain variable domains, is concentrated in three segments known as complementarity determining regions (CDRs) or hypervariable loops (HVLs). CDRs are defined by sequence comparison in Kabat et al., 1991, In: Sequences of Proteins of Immunological Interest, 5th Ed. Public Health Service, National Institutes of Health, Bethesda, Md., whereas HVLs are structurally defined according to the three-dimensional structure of the variable domain, as described by Chothia and Lesk, 1987, J. Mol. Biol. 196: 901-917. Where these two methods result in slightly different identifications of a CDR, the structural definition is preferred. As defined by Kabat, CDR-L1 is positioned at about residues 24-34, CDR-L2, at about residues 50-56, and CDR-L3, at about residues 89-97 in the light chain variable domain; CDR-H1 is positioned at about residues 31-35, CDR-H2 at about residues 50-65, and CDR-H3 at about residues 95-102 in the heavy chain variable domain. The CDR1, CDR2, CDR3 of the heavy and light chains therefore define the unique and functional properties specific for a given antibody.

The three CDRs within each of the heavy and light chains are separated by framework regions (FR), which contain sequences that tend to be less variable. From the amino terminus to the carboxy terminus of the heavy and light chain variable domains, the FRs and CDRs are arranged in the order: FR1, CDR1, FR2, CDR2, FR3, CDR3, and FR4. The largely β-sheet configuration of the FRs brings the CDRs within each of the chains into close proximity to each other as well as to the CDRs from the other chain. The resulting conformation contributes to the antigen binding site (see Kabat et al., 1991, NIH Publ. No. 91-3242, Vol. I, pages 647-669), although not all CDR residues are necessarily directly involved in antigen binding.

FR residues and Ig constant domains are not directly involved in antigen binding, but contribute to antigen binding and/or mediate antibody effector function. Some FR residues are thought to have a significant effect on antigen binding in at least three ways: by noncovalently binding directly to an epitope, by interacting with one or more CDR residues, and by affecting the interface between the heavy and light chains. The constant domains are not directly involved in antigen binding but mediate various Ig effector functions.

The light chains of vertebrate immunoglobulins are assigned to one of two clearly distinct classes, kappa (κ) and lambda (λ), based on the amino acid sequence of the constant domain. By comparison, the heavy chains of mammalian immunoglobulins are assigned to one of five major classes, according to the sequence of the constant domains: IgA, IgD, IgE, IgG, and IgM. IgG and IgA are further divided into subclasses (isotypes), e.g., IgG₁, IgG₂, IgG₃, IgG₄, IgA₁, and IgA₂. The heavy chain constant domains that correspond to the different classes of immunoglobulins are called α, δ, ε, γ, and µ, respectively. The subunit structures and three-dimensional configurations of the classes of native immunoglobulins are well known.

The terms, "antibody", "anti-CD40 antibody", "humanized anti-CD40 antibody", and "variant humanized anti-CD40 antibody" are used herein in the broadest sense and specifically encompass monoclonal antibodies (including full length monoclonal antibodies), polyclonal antibodies, multispecific antibodies (e.g., bispecific antibodies), and antibody fragments such as variable domains and other portions of antibodies that exhibit a desired biological activity, e.g., CD40 binding.

The term "monoclonal antibody" (mAb) refers to an antibody of a population of substantially homogeneous antibodies; that is, the individual antibodies in that population are identical except for naturally occurring mutations that may be present in minor amounts. Monoclonal antibodies are highly specific, being directed against a single antigenic determinant, an "epitope". Therefore, the modifier "monoclonal" is indicative of a substantially homogeneous population of antibodies directed to the identical epitope and is not to be construed as requiring production of the antibody by any particular method. It should be understood that monoclonal antibodies can be made by any technique or methodology known in the art; including e.g., the hybridoma method ( Kohler et al., 1975, Nature 256:495), or recombinant DNA methods known in the art (see, e.g., U.S. Pat. No. 4,816,567), or methods of isolation of monoclonal recombinantly produced using phage antibody libraries, using techniques described in Clackson et al., 1991, Nature 352: 624-628, and Marks et al., 1991, J. Mol. Biol. 222: 581-597.

Chimeric antibodies consist of the heavy and light chain variable regions of an antibody from one species (e.g., a non-human mammal such as a mouse) and the heavy and light chain constant regions of another species (e.g., human) antibody and can be obtained by linking the DNA sequences encoding the variable regions of the antibody from the first species (e.g., mouse) to the DNA sequences for the constant regions of the antibody from the second (e.g. human) species and transforming a host with an expression vector containing the linked sequences to allow it to produce a chimeric antibody. Alternatively, the chimeric antibody also could be one in which one or more regions or domains of the heavy and/or light chain is identical with, homologous to, or a variant of the corresponding sequence in a monoclonal antibody from another immunoglobulin class or isotype, or from a consensus or germline sequence. Chimeric antibodies can include fragments of such antibodies, provided that the antibody fragment exhibits the desired biological activity of its parent antibody, for example binding to the same epitope (see, e.g., U.S. Pat. No. 4,816,567; and Morrison et al., 1984, Proc. Natl. Acad. Sci. USA 81: 6851-6855).

The terms, "antibody fragment", "anti-CD40 antibody fragment", "humanized anti-CD40 antibody fragment", "variant humanized anti-CD40 antibody fragment" refer to a portion of a full length anti-CD40 antibody, in which a variable region or a functional capability is retained, for example, specific CD40 epitope binding. Examples of antibody fragments include, but are not limited to, a Fab, Fab', F(ab')₂, Fd, Fv, scFv and scFv-Fc fragment, a diabody, a linear antibody, a single-chain antibody, a minibody, a diabody formed from antibody fragments, and multispecific antibodies formed from antibody fragments.

Full length antibodies can be treated with enzymes such as papain or pepsin to generate useful antibody fragments. Papain digestion is used to produces two identical antigen-binding antibody fragments called "Fab" fragments, each with a single antigen-binding site, and a residual "Fc" fragment. The Fab fragment also contains the constant domain of the light chain and the C_{H1} domain of the heavy chain. Pepsin treatment yields a F(ab')₂ fragment that has two antigen-binding sites and is still capable of cross-linking antigen.

Fab' fragments differ from Fab fragments by the presence of additional residues including one or more cysteines from the antibody hinge region at the C-terminus of the C_{H1} domain. F(ab')₂ antibody fragments are pairs of Fab' fragments linked by cysteine residues in the hinge region. Other chemical couplings of antibody fragments are also known.

"Fv" fragment is contains a complete antigen-recognition and binding site consisting of a dimer of one heavy and one light chain variable domain in tight, noncovalent association. In this configuration, the three CDRs of each variable domain interact to define an antigen-biding site on the surface of the V_{H}-V_{L} dimer. Collectively, the six CDRs confer antigen-binding specificity to the antibody.

A "single-chain Fv" or "scFv" antibody fragment is a single chain Fv variant comprising the V_{H} and V_{L} domains of an antibody where the domains are present in a single polypeptide chain. The single chain Fv is capable of recognizing and binding antigen. The scFv polypeptide may optionally also contain a polypeptide linker positioned between the V_{H} and V_{L} domains in order to facilitate formation of a desired three-dimensional structure for antigen binding by the scFv (see, e.g., Pluckthun, 1994, In The Pharmacology of monoclonal Antibodies, Vol. 113, Rosenburg and Moore eds., Springer-Verlag, New York, pp. 269-315).

Other recognized antibody fragments include those that comprise a pair of tandem Fd segments (V_{H}-C_{H1}-V_{H}-C_{H1}) to form a pair of antigen binding regions. These "linear antibodies" can be bispecific or monospecific as described in, for example, Zapata et al. 1995, Protein Eng. 8(10):1057-1062.

A humanized antibody or a humanized antibody fragment is a specific type of chimeric antibody which includes an immunoglobulin amino acid sequence variant, or fragment thereof, which is capable of binding to a predetermined antigen and which, comprises one or more FRs having substantially the amino acid sequence of a human immunoglobulin and one or more CDRs having substantially the amino acid sequence of a non-human immunoglobulin. This non-human amino acid sequence often referred to as an "import" sequence is typically taken from an "import" antibody domain, particularly a variable domain. In general, a humanized antibody includes at least the CDRs or HVLs of a non-human antibody, inserted between the FRs of a human heavy or light chain variable domain. The present disclosure describes specific humanized anti-CD40 antibodies which contain CDRs derived from the murine monoclonal antibodies shown in Tables 3 and 4 inserted between the FRs of human germline sequence heavy and light chain variable domains. It will be understood that certain murine FR residues may be important to the function of the humanized antibodies and therefore certain of the human germline sequence heavy and light chain variable domains residues are modified to be the same as those of the corresponding murine sequence.

In another aspect, a humanized anti-CD40 antibody comprises substantially all of at least one, and typically two, variable domains (such as contained, for example, in Fab, Fab', F(ab')2, Fabc, and Fv fragments) in which all, or substantially all, of the CDRs correspond to those of a non-human immunoglobulin, and specifically herein, all of the CDRs are murine sequences as detailed in Tables 1 through 4 herein below and all, or substantially all, of the FRs are those of a human immunoglobulin consensus or germline sequence. In another aspect, a humanized anti-CD40 antibody also includes at least a portion of an immunoglobulin Fc region, typically that of a human immunoglobulin. Ordinarily, the antibody will contain both the light chain as well as at least the variable domain of a heavy chain. The antibody also may include one or more of the C_{H1}, hinge, C_{H2}, C_{H3}, and/or C_{H4} regions of the heavy chain, as appropriate.

A humanized anti-CD40 antibody can be selected from any class of immunoglobulins, including IgM, IgG, IgD, IgA and IgE, and any isotype, including IgG₁, IgG₂, IgG₃, IgG₄, IgA₁ and IgA₂. An alternative humanized anti-CD40 antibody can comprise sequences from more than one immunoglobulin class or isotype, and selecting particular constant domains to optimize desired effector functions is within the ordinary skill in the art. In specific embodiments, the present invention provides antibodies that are IgG1 antibodies and more particularly, are IgG1 antibodies in which there is a knock-out of effector functions.

The FRs and CDRs, or HVLs, of a humanized anti-CD40 antibody need not correspond precisely to the parental sequences. For example, one or more residues in the import CDR, or HVL, or the consensus or germline FR sequence may be altered (e.g., mutagenized) by substitution, insertion or deletion such that the resulting amino acid residue is no longer identical to the original residue in the corresponding position in either parental sequence but the antibody nevertheless retains the function of binding to CD40. Such alteration typically will not be extensive and will be conservative alterations. Usually, at least 75% of the humanized antibody residues will correspond to those of the parental consensus or germline FR and import CDR sequences, more often at least 90%, and most frequently greater than 95%, or greater than 98% or greater than 99%.

Immunoglobulin residues that affect the interface between heavy and light chain variable regions ("the V_{L}-V_{H} interface") are those that affect the proximity or orientation of the two chains with respect to one another. Certain residues that may be involved in interchain interactions include V_{L} residues 34, 36, 38, 44, 46, 87, 89, 91, 96, and 98 and V_{H} residues 35, 37, 39, 45, 47, 91, 93, 95, 100, and 103 (utilizing the numbering system set forth in Kabat et al., Sequences of Proteins of Immunological Interest (National Institutes of Health, Bethesda, Md., 1987)). U.S. Pat. No. 6,407,213 also discusses that residues such as V_{L} residues 43 and 85, and V_{H} residues 43 and 60 also may be involved in this interaction. While these residues are indicated for human IgG only, they are applicable across species. Important antibody residues that are reasonably expected to be involved in interchain interactions are selected for substitution into the consensus sequence.

The terms "consensus sequence" and "consensus antibody" refer to an amino acid sequence which comprises the most frequently occurring amino acid residue at each location in all immunoglobulins of any particular class, isotype, or subunit structure, e.g., a human immunoglobulin variable domain. The consensus sequence may be based on immunoglobulins of a particular species or of many species. A "consensus" sequence, structure, or antibody is understood to encompass a consensus human sequence as described in certain embodiments, and to refer to an amino acid sequence which comprises the most frequently occurring amino acid residues at each location in all human immunoglobulins of any particular class, isotype, or subunit structure. Thus, the consensus sequence contains an amino acid sequence having at each position an amino acid that is present in one or more known immunoglobulins, but which may not exactly duplicate the entire amino acid sequence of any single immunoglobulin. The variable region consensus sequence is not obtained from any naturally produced antibody or immunoglobulin. Kabat et al., 1991, Sequences of Proteins of Immunological Interest, 5th Ed. Public Health Service, National Institutes of Health, Bethesda, Md., and variants thereof. The FRs of heavy and light chain consensus sequences, and variants thereof, provide useful sequences for the preparation of humanized anti-CD40 antibodies. See, for example, U.S. Pat. Nos. 6,037,454 and 6,054,297.

Human germline sequences are found naturally in human population. A combination of those germline genes generates antibody diversity. Germline antibody sequences for the light chain of the antibody come from conserved human germline kappa or lambda v-genes and j-genes. Similarly the heavy chain sequences come from germline v-, d- and j-genes (LeFranc, M-P, and LeFranc, G, "The Immunoglobulin Facts Book" Academic Press, 2001).

As used herein, "variant", "anti-CD40 variant", "humanized anti-CD40 variant", or "variant humanized anti-CD40" each refers to a humanized anti-CD40 antibody having at least a heavy chain variable murine CDR from any of the sequences of SEQ ID NO: 1 through 4 or a light chain murine CDR sequence derived from the murine monoclonal antibody as shown in any of SEQ ID NO:5 through SEQ ID NO:8 and FR sequences derived from human consensus sequences. Variants include those having one or more amino acid changes in one or both light chain or heavy chain variable domains, provided that the amino acid change does not substantially impair binding of the antibody to CD40. Exemplary humanized antibodies produced herein include those designated as Antibody A, Antibody B and Antibody C and the various heavy and light chain sequences of the same are shown in SEQ ID NOs 26 through SEQ ID NO:40.

An "isolated" antibody is one that has been identified and separated and/or recovered from a component of its natural environment. Contaminant components of the antibody's natural environment are those materials that may interfere with diagnostic or therapeutic uses of the antibody, and can be enzymes, hormones, or other proteinaceous or nonproteinaceous solutes. In one aspect, the antibody will be purified to at least greater than 95% isolation by weight of antibody.

An isolated antibody includes an antibody in situ within recombinant cells in which it is produced, since at least one component of the antibody's natural environment will not be present. Ordinarily however, an isolated antibody will be prepared by at least one purification step in which the recombinant cellular material is removed.

The term "antibody performance" refers to factors that contribute to antibody recognition of antigen or the effectiveness of an antibody in vivo. Changes in the amino acid sequence of an antibody can affect antibody properties such as folding, and can influence physical factors such as initial rate of antibody binding to antigen (kₐ), dissociation constant of the antibody from antigen (k_{d}), affinity constant of the antibody for the antigen (Kd), conformation of the antibody, protein stability, and half-life of the antibody.

The term "epitope tagged" when used herein, refers to an anti-CD40 antibody fused to an "epitope tag". An "epitope tag" is a polypeptide having a sufficient number of amino acids to provide an epitope for antibody production, yet is designed such that it does not interfere with the desired activity of the humanized anti-CD40 antibody. The epitope tag is usually sufficiently unique such that an antibody raised against the epitope tag does not substantially cross-react with other epitopes. Suitable tag polypeptides generally contain at least 6 amino acid residues and usually contain about 8 to 50 amino acid residues, or about 9 to 30 residues. Examples of epitope tags and the antibody that binds the epitope include the flu HA tag polypeptide and its antibody 12CA5 (Field et al., 1988 Mol. Cell. Biol. 8: 2159-2165; c-myc tag and 8F9, 3C7, 6E10, G4, B7 and 9E10 antibodies thereto (Evan et al., 1985, Mol. Cell. Biol. 5(12):3610-3616; and Herpes simplex virus glycoprotein D (gD) tag and its antibody (Paborsky et al. 1990, Protein Engineering 3(6): 547-553). In certain embodiments, the epitope tag is a "salvage receptor binding epitope". As used herein, the term "salvage receptor binding epitope" refers to an epitope of the Fc region of an IgG molecule (such as IgG₁, IgG₂, IgG₃, or IgG₄) that is responsible for increasing the in vivo serum half-life of the IgG molecule.

The antibodies also may be conjugated to prodrugs. A "prodrug" is a precursor or derivative form of a pharmaceutically active substance. See, for example, Wilman, 1986, "Prodrugs in Cancer Chemotherapy", In Biochemical Society Transactions, 14, pp. 375-382, 615th Meeting Belfast and Stella et al., 1985, "Prodrugs: A Chemical Approach to Targeted Drug Delivery, In: "Directed Drug Delivery, Borchardt et al., (ed.), pp. 247-267, Humana Press.

For diagnostic as well as therapeutic monitoring purposes, the antibodies used in the method of the invention also may be conjugated to a label, either a label alone or a label and an additional second agent (prodrug and the like). A label, as distinguished from the other second agents refers to an agent that is a detectable compound or composition and it may be conjugated directly or indirectly to a humanized antibody of the present invention. The label may itself be detectable (e.g., radioisotope labels or fluorescent labels) or, in the case of an enzymatic label, may catalyze chemical alteration of a substrate compound or composition that is detectable. Labeled humanized anti-CD40 antibody can be prepared and used in various applications including in vitro and in vivo diagnostics.

The antibodies used in the method of the present invention may be formulated as part of a liposomal preparation in order to effect delivery thereof in vivo. A "liposome" is a small vesicle composed of various types of lipids, phospholipids, and/or surfactant. Liposomes are useful for delivery to a mammal of a compound or formulation, such as a humanized anti-CD40 antibody disclosed herein, optionally, coupled to or in combination with one or more pharmaceutically active agents and/or labels. The components of the liposome are commonly arranged in a bilayer formation, similar to the lipid arrangement of biological membranes.

The term "mammal" for purposes of treatment refers to any animal classified as a mammal, including humans, domesticated and farm animals, and zoo, sports, or pet animals, such as dogs, horses, cats, cows, and the like. Preferably, the mammal is human.

A "disorder", as used herein refers to lupus nephritis.

The term "intravenous bolus" or "intravenous push" refers to drug administration into a vein of an animal or human such that the body receives the drug in approximately 15 minutes or less, generally 5 minutes or less.

The term "subcutaneous administration" refers to introduction of an agent under the skin of an animal or human patient, preferable within a pocket between the skin and underlying tissue, by relatively slow, sustained delivery from a drug receptacle. Pinching or drawing the skin up and away from underlying tissue may create the pocket.

The term "subcutaneous infusion" refers to introduction of a drug under the skin of an animal or human patient, preferably within a pocket between the skin and underlying tissue, by relatively slow, sustained delivery from a drug receptacle for a period of time including, but not limited to, 30 minutes or less, or 90 minutes or less. Optionally, the infusion may be made by subcutaneous implantation of a drug delivery pump implanted under the skin of the animal or human patient, wherein the pump delivers a predetermined amount of drug for a predetermined period of time, such as 30 minutes, 90 minutes, or a time period spanning the length of the treatment regimen.

The term "subcutaneous bolus" refers to drug administration beneath the skin of an animal or human patient, where bolus drug delivery is less than approximately 15 minutes; in another aspect, less than 5 minutes, and in still another aspect, less than 60 seconds. In yet even another aspect, administration is within a pocket between the skin and underlying tissue, where the pocket may be created by pinching or drawing the skin up and away from underlying tissue.

The term "therapeutically effective amount" is used to refer to an amount of an active agent that relieves or ameliorates one or more of the symptoms of the disorder being treated. In doing so it is that amount that has a beneficial patient outcome, for example, a growth arrest effect or causes the deletion of the cell. In one aspect, the therapeutically effective amount has apoptotic activity, or is capable of inducing cell death. In another aspect, the therapeutically effective amount refers to a target serum concentration that has been shown to be effective in, for example, slowing disease progression. Efficacy can be measured in conventional ways, depending on the condition to be treated. For example, in neoplastic diseases or disorders characterized by cells expressing CD40, efficacy can be measured by assessing the time to disease progression, or determining the response rates.

The terms "treatment" and "therapy" and the like, as used herein, are meant to include therapeutic as well as prophylactic, or suppressive measures for a disease or disorder leading to any clinically desirable or beneficial effect, including but not limited to alleviation or relief of one or more symptoms, regression, slowing or cessation of progression of the disease or disorder. Thus, for example, the term treatment includes the administration of an agent prior to or following the onset of a symptom of a disease or disorder thereby preventing or removing one or more signs of the disease or disorder. As another example, the term includes the administration of an agent after clinical manifestation of the disease to combat the symptoms of the disease. Further, administration of an agent after onset and after clinical symptoms have developed where administration affects clinical parameters of the disease or disorder, such as the degree of tissue injury or the amount or extent of metastasis, whether or not the treatment leads to amelioration of the disease, comprises "treatment" or "therapy" as used herein. Moreover, as long as the compositions of the invention either alone or in combination with another therapeutic agent alleviate or ameliorate at least one symptom of a disorder being treated as compared to that symptom in the absence of use of the humanized CD40 antibody composition, the result should be considered an effective treatment of the underlying disorder regardless of whether all the symptoms of the disorder are alleviated or not.

The term "package insert" is used to refer to instructions customarily included in commercial packages of therapeutic products, that contain information about the indications, usage, administration, contraindications and/or warnings concerning the use of such therapeutic products.

Unless otherwise defined herein, the terms "pharmaceutical composition," pharmaceutical formulation" and "formulation" can be used interchangeably.

### Antibodies

The humanized anti-CD40 antibodies and binding agents can be used in the treatment and/or prevention a variety of diseases or disorders characterized by the proliferation of cells expressing the CD40 surface antigen such as lupus nephritis. A humanized anti-CD40 antibody and a CD40 binding agent each includes at least a portion that specifically recognizes a CD40 epitope (i.e., an antigen-binding fragment).

Methods for making the anti-CD40 antibodies have been previously described in US20110243932.

As previously described in US20110243932, the initial characterization murine antibodies were selected based on CD40 binding characterization.

From these initial studies, murine antibodies were selected that had the following heavy chain variable regions shown in Table 1 and the light chain variable regions shown in Table 2:

**Table 1: CD40 Murine Leads - VH Sequences**

| | |
|---|---|
| 2H11 | EVQLQQSGAELVRPGASVKLSCTASGFNIKDYYVHWVKQRPEKGLEWIGR IDPEDGDSKYAPKFQGKATMTADTSSNTAYLHLSSLTSEDTAVYYCTTSY YVGTYGYWGQGTTLTVSS (SEQ ID NO:1) |
| 10F2 | EVQLQQSGAELVRPGASVKLSCTASGFNIKDYYIHWVKQRPEKGLEWIGR IDPEDGDTKYDPKFQGKATMTADTSSNTAYLHLSSLTSEDTAVYYCTTSY YVGTYGYWGQGTTLTVSS(SEQ ID NO:2) |
| 19B10 | EVQLQQSGAELVRPGASVQLSCTASGFNIKDYYVHWVKQRPEKGLEWIGR IDPEDGDTKFAPKFQGKATMTADTSSNTVYLHLSSLTSEDTAVYYCTTSY YVGTYGYWGQGTTLTVSS(SEQ ID NO:3) |
| 20E2 | EVQLVESGGGLVKPGGSRKLSCAASGFTFSDYGMHWVRQAPEKGLEWVAY ISSGNRIIYYADTVKGRFTISRDNAKNTLFLQMTSLRSEDTALYYCARQD GYRYAMDYWGQGTSVTVSS(SEQ ID NO:4) |

**TABLE 2: CD40 Murine Leads - VK Sequences**

| | |
|---|---|
| 2H11 | QIVLTQSPAIMSASPGEKVTITCSASSSVSYMLWFQQKPGTSPKLWIYST SNLASGVPARFGGSGSGTSYSLTISRMEAEDAATYYCQQRTFYPYTFGGG TKLEIK (SEQ ID NO:5) |
| 10F2 | QIVLTQSPTIMSASPGEKVIITCSATSSVSYILWFQQKPGTSPKLWIYST SNLASGVPARFSGSGSGASYSLTISRMEAEDAATYYCQQRTFYPYTFGGG TKLEIK (SEQ ID NO:6) |
| 19B10 | QIVLTQSPAIMSASPGEKVTITCSASSSVSYMLWFQQKPGTSPKLWIYST SNLASGVPARFSGSGSGTSYSLTISRMEAEDAATYYCQQRTFYPYTFGGG TKLEIK (SEQ ID NO:7) |
| 20E2 | DIVMTQSPSSLTVTAGEKVTMSCKSSQSLLNSGNQKNYLTWHQQKPGQPP KLLIYWTSTRESGVPDRFTGSGSGTDFTLTISNLQAEDLAVYYCQNDYTY PLTFGAGTKLELK (SEQ ID NO:8) |

Human framework sequences were selected for each of the mouse leads based on the framework homology, CDR structure, conserved canonical residues, conserved interface packing residues and other parameters.

The murine heavy chain and light chain CDRs of the various murine antibodies selected antibodies are shown in Table 3 and Table 4, respectively:

**Table 3:**

| **HEAVY CHAIN CDR sequences** | | | |
|---|---|---|---|
| **Construct name** | **H-CDR1** | **H-CDR2** | **H-CDR3** |
| 2H11 | GFNIK***DYYVH*** | ***RIDPEDGDSKYAPKFQG*** | ***SYYVGTYGY*** |
| | **SEQ ID NO:9** | **SEQ ID NO:12** | **SEQ ID NO:16** |
| 10F2 | GFNIK***DYYIH*** | ***RIDPEDGDTKYDPKFQG*** | ***SYYVGTYGY*** |
| 19B10 | **SEQ ID NO:10** | **SEQ ID NO:13** | **SEQ ID NO:16** |
| | GFNIK***DYYVH*** | ***RIDPEDGDTKFAPKFQG*** | ***SYYVGTYGY*** |
| 20E2 | **SEQ ID NO:9** | **SEQ ID NO:14** | **SEQ ID NO:16** |
| | GFTFS***DYGMH*** | ***YISSGNRIIYYADTVKG*** | ***QDGYRYAMDY*** |
| | **SEQ ID NO:11** | **SEQ ID NO:15** | **SEQ ID NO:17** |

The H-CDR1 listed above is using the sequence using the Chothia numbering system (Al-Lazikani et al., (1997) JMB 273,927-948). The Kabats numbering for the sequences is denoted by the bold italicized text and the IMGT numbering is shown by underlined text of the residues in the above table for CDR1 and CDR2. The sequences for the H-CDR3 for each of 2H11, 10F2 and 19B10 is TTSYYVGTYGY (SEQ ID NO:77) and for 20E2 is ARQDGYRYAMDY (SEQ ID NO:78).

**Table 4:**

| **LIGHT CHAIN CDR sequences** | | | |
|---|---|---|---|
| **Construct name** | **L-CDR1** | **L-CDR2** | **L-CDR3** |
| 2H11 | ***SASSSVSYML*** | ***STSNLAS*** | ***QQRTFYPYT*** |
| 10F2 | **SEQ ID NO:18** | **SEQ ID NO:22** | SEQ **ID NO:24** |
| | ***SATSSVSYIL*** | ***STSNLAS*** | ***QQRTFYPYT*** |
| 19B10 | **SEQ ID NO:19** | **SEQ ID NO:22** | **SEQ ID NO:24** |
| | ***SASSSVSYML*** | ***STSNLAS*** | ***QQRTFYPYT*** |
| 20E2 | **SEQ ID NO:20** | **SEQ ID NO:22** | **SEQ ID NO:24** |
| | ***KSSQSLLNSGNQKNYLT*** | ***WTSTRES*** | ***QNDYTYPLT*** |
| | **SEQ ID NO:21** | **SEQ ID NO:23** | **SEQ ID NO:25** |

Again, the Chothia numbering system is used in Table 4 with the Kabats numbering for the sequences being denoted by the bold, italicized text and the IMGT numbering is shown by underlined text.

Fabs that showed better or equal binding as compared to the chimeric parent Fab were selected for conversion to IgG. Clones from the 20E2 series were converted to two different IgG formats: a) IgG4DM (double mutant) has two mutations in the Fc / hinge region, Ser228Pro which reduces half-molecule formation and Leu235Glu which further reduces FcγR binding. b) IgG1KO (knock-out of effector functions) has two mutations in the Fc region, Leu234Ala and Leu235Ala, which reduce effector function such as FcyR and complement binding. Both IgG formats are described in the literature. Example 1 describes the humanization of three candidates in further detail. The results of such humanization resulted in humanized antibody sequences, which have the heavy and light chain sequences shown below:

| Identity | Sequence | SEQ ID NO: |
|---|---|---|
| **Antibody A (Light Chain)** | DIVMTQSPDSLAVSLGERATMSCKSSQSLLNSGNQKNYLTW HQQKPGQPPKLLIYWTSTRESGVPDRFSGSGSGTDFTLTIS SLQAEDVAVYYCQNDYTYPLTFGGGTKVEIKRTVAAPSVFI FPPSDEQLKSGTASVVCLLNNFYPREAKVQWKVDNALQSGN SQESVTEQDSKDSTYSLSSTLTLSKADYEKHKVYACEVTHQ GLSSPVTKSFNRGEC | 26 |
| **Antibody A (Heavy Chain, IgG1KO)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTALYYCARQDGYRYAMDYWAQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSREEMTKNQVSLTC LVKGFYPSDIAVEWESNGQPENNYKTTPPVLDSDGSFFLYS KLTVDKSRWQQGNVFSCSVMHEALHNHYTQKSLSLSPGK | 27 |
| **Antibody A (Heavy Chain, IgG1)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTALYYCARQDGYRYAMDYWAQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPELLGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSREEMTKNQVSLTC LVKGFYPSDIAVEWESNGQPENNYKTTPPVLDSDGSFFLYS KLTVDKSRWQQGNVFSCSVMHEALHNHYTQKSLSLSPGK | 28 |
| **Antibody A (Heavy Chain, IgG4DM)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTALYYCARQDGYRYAMDYWAQGTLVTVSSASTK GPSVFPLAPCSRSTSESTAALGCLVKDYFPEPVTVSWNSGA LTSGVHTFPAVLQSSGLYSLSSVVTVPSSSLGTKTYTCNVD HKPSNTKVDKRVESKYGPPCPPCPAPEFEGGPSVFLFPPKP KDTLMISRTPEVTCVVVDVSQEDPEVQFNWYVDGVEVHNAK TKPREEQFNSTYRVVSVLTVLHQDWLNGKEYKCKVSNKGLP | 29 |
| | SSIEKTISKAKGQPREPQVYTLPPSQEEMTKNQVSLTCLVK GFYPSDIAVEWESNGQPENNYKTTPPVLDSDGSFFLYSRLT VDKSRWQEGNVFSCSVMHEALHNHYTQKSLSLSLGK | |
| **Antibody A (Heavy, IgGlKOb)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTALYYCARQDGYRYAMDYWAQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSRDELTKNQVSLTC LVKGFYPSDIAVEWESNGQPENNYKTTPPVLDSDGSFFLYS KLTVDKSRWQQGNVFSCSVMHEALHNHYTQKSLSLSPGK | 30 |
| **Antibody B (Light Chain)** | DIVMTQSPDSLAVSLGEKVTINCKSSQSLLNSGNQKNYL TWHQQKPGQPPKLLIYWTSTRESGVPDRFSGSGSGTDFT LTISSLQAEDVAVYYCQNDYTYPLTFGGGTKVEIKRTVA APSVFIFPPSDEQLKSGTASVVCLLNNFYPREAKVQWKV DNALQSGNSQESVTEQDSKDSTYSLSSTLTLSKADYEKH KVYACEVTHQGLSSPVTKSFNRGEC | 31 |
| **Antibody B (Heavy Chain, IgG1KO)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTAVYYCARQDGYRYAMDYWGQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSREEMTKNQVSLTC LVKGFYPSDIAVEWESNGQPENNYKTTPPVLDSDGSFFLYS KLTVDKSRWQQGNVFSCSVMHEALHNHYTQKSLSLSPGK | 32 |
| **Antibody B (Heavy Chain, IgG1)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTAVYYCARQDGYRYAMDYWGQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPELLGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSREEMTKNQVSLTC LVKGFYPSDIAVEWESNGQPENNYKTTPPVLDSDGSFFLYS KLTVDKSRWQQGNVFSCSVMHEALHNHYTQKSLSLSPGK | 33 |
| **Antibody B (Heavy Chain,IgG4 DM)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTAVYYCARQDGYRYAMDYWGQGTLVTVSSASTK GPSVFPLAPCSRSTSESTAALGCLVKDYFPEPVTVSWNSGA LTSGVHTFPAVLQSSGLYSLSSVVTVPSSSLGTKTYTCNVD HKPSNTKVDKRVESKYGPPCPPCPAPEFEGGPSVFLFPPKP KDTLMISRTPEVTCVVVDVSQEDPEVQFNWYVDGVEVHNAK TKPREEQFNSTYRVVSVLTVLHQDWLNGKEYKCKVSNKGLP SSIEKTISKAKGQPREPQVYTLPPSQEEMTKNQVSLTCLVK GFYPSDIAVEWESNGQPENNYKTTPPVLDSDGSFFLYSRLT | 34 |
| | VDKSRWQEGNVFSCSVMHEALHNHYTQKSLSLSLGK | |
| **Antibody B (Heavy Chain, IgGIKOb)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTAVYYCARQDGYRYAMDYWGQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSRDELTKNQVSLTC LVKGFYPSDIAVEWESNGQPENNYKTTPPVLDSDGSFFLYS KLTVDKSRWQQGNVFSCSVMHEALHNHYTQKSLSLSPGK | 35 |
| **Antibody C (Light Chain)** | DIQMTQSPSSLSASVGDRVTITCSASSSVSYMLWFQ QKPGKAPKLLIYSTSNLASGVPSRFSGSGSGTDFTL TISSLQPEDFATYYCQQRTFYPYTFGGGTKVEIKRT VAAPSVFIFPPSDEQLKSGTASVVCLLNNFYPREAK VQWKVDNALQSGNSQESVTEQDSKDSTYSLSSTLTL SKADYEKHKVYACEVTHQGLSSPVTKSFNRGEC | 36 |
| **Antibody C (Heavy Chain, IgG1KO)** | QVQLVQSGAEVKKPGASVKVSCTASGFNIKDYYVHWVKQAP GQGLEWMGRIDPEDGDSKYAPKFQGKATMTADTSTSTVYME LSSLRSEDTAVYYCTTSYYVGTYGYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKRVEPKSCDKTHTCPPCPAPEAAGGPSVFLFPP KPKDTLMISRTPEVTCVVVDVSHEDPEVKFNWYVDGVEVHN AKTKPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYSK LTVDKSRWQQGNVFSCSVMHEALHNHYTQKSLSLSPGK | 37 |
| **Antibody C (Heavy Chain, IgG1)** | QVQLVQSGAEVKKPGASVKVSCTASGFNIKDYYVHWVKQAP GQGLEWMGRIDPEDGDSKYAPKFQGKATMTADTSTSTVYME LSSLRSEDTAVYYCTTSYYVGTYGYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKRVEPKSCDKTHTCPPCPAPELLGGPSVFLFPP KPKDTLMISRTPEVTCVVVDVSHEDPEVKFNWYVDGVEVHN AKTKPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYSK LTVDKSRWQQGNVFSCSVMHEALHNHYTQKSLSLSPGK | 38 |
| **Antibody C (Heavy Chain,IgG4 DM)** | QVQLVQSGAEVKKPGASVKVSCTASGFNIKDYYVHWVKQAP GQGLEWMGRIDPEDGDSKYAPKFQGKATMTADTSTSTVYME LSSLRSEDTAVYYCTTSYYVGTYGYWGQGTLVTVSSASTKG PSVFPLAPCSRSTSESTAALGCLVKDYFPEPVTVSWNSGAL TSGVHTFPAVLQSSGLYSLSSVVTVPSSSLGTKTYTCNVDH KPSNTKVDKRVESKYGPPCPPCPAPEFEGGPSVFLFPPKPK DTLMISRTPEVTCVVVDVSQEDPEVQFNWYVDGVEVHNAKT KPREEQFNSTYRVVSVLTVLHQDWLNGKEYKCKVSNKGLPS SIEKTISKAKGQPREPQVYTLPPSQEEMTKNQVSLTCLVKG FYPSDIAVEWESNGQPENNYKTTPPVLDSDGSFFLYSRLTV DKSRWQEGNVFSCSVMHEALHNHYTQKSLSLSLGK | 39 |
| | | |
| **Antibody C (Heavy Chain, IgGIKOb)** | QVQLVQSGAEVKKPGASVKVSCTASGFNIKDYYVHWVKQAP GQGLEWMGRIDPEDGDSKYAPKFQGKATMTADTSTSTVYME LSSLRSEDTAVYYCTTSYYVGTYGYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKRVEPKSCDKTHTCPPCPAPEAAGGPSVFLFPP KPKDTLMISRTPEVTCVVVDVSHEDPEVKFNWYVDGVEVHN AKTKPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYSK LTVDKSRWQQGNVFSCSVMHEALHNHYTQKSLSLSPGK | 40 |

The humanized anti-CD40 antibodies described herein, including antigen-binding fragments thereof, such as heavy and light chain variable domains, comprise an amino acid sequence of the residues derived from the CDRs Antibody A (heavy chain sequence = SEQ ID NO:27; SEQ ID NO:28; SEQ ID NO:29 or SEQ ID NO:30; light chain sequence = SEQ ID NO:26), Antibody B (heavy chain sequence = SEQ ID NO:32; SEQ ID NO:33; SEQ ID NO:34; or SEQ ID NO:35; light chain sequence = SEQ ID NO:31) and Antibody C (heavy chain sequence = SEQ ID NO:37; SEQ ID NO:38; SEQ ID NO:39 or SEQ ID NO:40; light chain sequence = SEQ ID NO:36;) described herein above and amino acid residues derived from framework regions of a human immunoglobulin. The humanized anti-CD40 antibodies optionally include specific amino acid substitutions in the consensus or germline framework regions.

The specific substitution of amino acid residues in these framework positions can improve various aspects of antibody performance including binding affinity and/or stability, over that demonstrated in humanized antibodies formed by "direct swap" of CDRs or HVLs into the human germline framework regions, as shown in the examples below.

Also described herein are other monoclonal antibodies with heavy chain (VH) sequences of SEQ ID NO:1 through SEQ ID NO:4 and light chain (VL) sequences of SEQ ID NO:5 to SEQ ID NO:8 (see Tables 1 and 2 above). The CDR sequence of these murine antibodies are shown in Tables 3 and 4 placing such CDRs into FRs of the human consensus heavy and light chain variable domains will yield useful humanized antibodies.

In some specific embodiments, the humanized anti-CD40 antibodies disclosed herein comprise at least a heavy or light chain variable domain comprising the CDRs or HVLs of the murine monoclonal antibodies as shown in Tables 1 through 4 above and the FRs of the human germline heavy and light chain variable domains. In exemplary embodiments, the humanized antibodies created herein are: Antibody A, Antibody B and Antibody C and the various heavy and light chain sequences of the same are shown in SEQ ID NOs 26 through SEQ ID NO:40.

In specific embodiments, antibodies are disclosed herein that have a heavy chain sequence of any of SEQ ID NO: 27, SEQ ID NO:28, SEQ ID NO:29 or SEQ ID NO:30 in combination with a light chain sequence of SEQ ID NO:26. Antibodies according to the invention include those that have a heavy chain sequence of SEQ ID NO:35, in combination with a light chain sequence of SEQ ID NO:31. In still additional embodiments, disclosed herein are humanized antibodies that have a heavy chain sequence of SEQ ID NO: 37, SEQ ID NO:38; SEQ ID NO:39 or SEQ ID NO: 40, in combination with a light chain sequence of SEQ ID NO:36.

The CDRs of these sequences are shown in Tables 3 and 4. In specific embodiments, it is contemplated that chimerical antibodies with switched CDR regions (i.e., for example switching one or two CDRs of Antibody A with the analogous CDR from Antibody C) between these exemplary immunoglobulins may yield useful antibodies.

In certain embodiments, the humanized anti-CD40 antibody is an antibody fragment. Various antibody fragments have been generally discussed above and there are techniques that have been developed for the production of antibody fragments. Fragments can be derived via proteolytic digestion of intact antibodies (see, e.g., Morimoto et al., 1992, Journal of Biochemical and Biophysical Methods 24:107-117; and Brennan et al., 1985, Science 229:81). Alternatively, the fragments can be produced directly in recombinant host cells. For example, Fab'-SH fragments can be directly recovered from E. coli and chemically coupled to form F(ab')₂ fragments (see, e.g., Carter et al., 1992, Bio/Technology 10:163-167). By another approach, F(ab')₂ fragments can be isolated directly from recombinant host cell culture. Other techniques for the production of antibody fragments will be apparent to the skilled practitioner.

Also described herein is a F(ab')₂ fragment of a humanized anti-CD40 antibody comprising a have a heavy chain sequence of any of SEQ ID NO:27, SEQ ID NO:28, SEQ ID NO:29 or SEQ ID NO:30 in combination with a light chain sequence of SEQ ID NO:26. Alternative antibodies include those that have a heavy chain sequence of SEQ ID NO:32, SEQ ID NO:33, SEQ ID NO:34 or SEQ ID NO:35, in combination with a light chain sequence of SEQ ID NO:31. In still additional embodiments, disclosed herein are humanized antibodies that have a heavy chain sequence of SEQ ID NO: 37, SEQ ID NO:38; SEQ ID NO:39 or SEQ ID NO: 40, in combination with a light chain sequence of SEQ ID NO:36. Such embodiments can include an intact antibody comprising such an F(ab')₂.

The effector domain of an antibody can be from any suitable vertebrate animal species and isotypes. The isotypes from different animal species differ in the abilities to mediate effector functions. For example, the ability of human immunoglobulin to mediate CDC and ADCC/ADCP is generally in the order of IgM≈IgG₁≈IgG₃>IgG₂>IgG₄ and IgG₁≈IgG₃>IgG₂/IgM/IgG₄, respectively. Murine immunoglobulins mediate CDC and ADCC/ADCP generally in the order of murine IgM≈IgG₃>>IgG_{2b}>IgG₂ₐ>>IgG₁ and IgG_{2b}>IgG₂ₐ>IgG₁>>IgG₃, respectively. In another example, murine IgG₂ₐ mediates ADCC while both murine IgG₂ₐ and IgM mediate CDC.

### Antibody Modifications

The humanized anti-CD40 antibodies and agents can include modifications of the humanized anti-CD40 antibody or antigen-binding fragment thereof.

Conjugates of the humanized anti-CD40 antibody can be made by known methods, using a variety of bifunctional protein coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., 1987, Science 238:1098. Carbon-14-labeled 1-isothiocyanatobenzyl-3-methyldiethylene triaminepentaacetic acid (MX-DTPA) is an exemplary chelating agent for conjugation of radionucleotide to the antibody. Conjugates also can be formed with a cleavable linker.

The humanized anti-CD40 antibodies disclosed herein can also be formulated as immunoliposomes. Liposomes containing the antibody are prepared by methods known in the art, such as described in Epstein et al., 1985, Proc. Natl. Acad. Sci. USA 82:3688; Hwang et al., 1980, Proc. Natl. Acad. Sci. USA 77:4030; and U.S. Pat. Nos. 4,485,045 and 4,544,545. Liposomes having enhanced circulation time are disclosed, for example, in U.S. Pat. No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of an antibody disclosed herein can be conjugated to the liposomes as described in Martin et al., 1982, J. Biol. Chem. 257:286-288 via a disulfide interchange reaction.

In other embodiments, covalent modifications of the humanized anti-CD40 antibody are also included. Covalent modifications include modification of cysteinyl residues, histidyl residues, lysinyl and amino-terminal residues, arginyl residues, tyrosyl residues, carboxyl side groups (aspartyl or glutamyl), glutaminyl and asparaginyl residues, or seryl, or threonyl residues. Another type of covalent modification involves chemically or enzymatically coupling glycosides to the antibody. Such modifications may be made by chemical synthesis or by enzymatic or chemical cleavage of the antibody, if applicable. Other types of covalent modifications of the antibody can be introduced into the molecule by reacting targeted amino acid residues of the antibody with an organic derivatizing agent that is capable of reacting with selected side chains or the amino- or carboxy-terminal residues.

Removal of any carbohydrate moieties present on the antibody can be accomplished chemically or enzymatically. Chemical deglycosylation is described by Hakimuddin et al., 1987, Arch. Biochem. Biophys. 259:52 and by Edge et al., 1981, Anal. Biochem., 118:131. Enzymatic cleavage of carbohydrate moieties on antibodies can be achieved by the use of a variety of endo- and exo-glycosidases as described by Thotakura et al., 1987, Meth. Enzymol 138:350.

Another type of useful covalent modification comprises linking the antibody to one of a variety of nonproteinaceous polymers, e.g., polyethylene glycol, polypropylene glycol, or polyoxyalkylenes, in the manner set forth in one or more of U.S. Pat. No. 4,640,835, U.S. Pat. No. 4,496,689, U.S. Pat. No. 4,301,144, U.S. Pat. No. 4,670,417, U.S. Pat. No. 4,791,192 and U.S. Pat. No. 4,179,337.

### Humanization and Amino Acid Sequence Variants

Amino acid sequence variants of the anti-CD40 antibody can be prepared by introducing appropriate nucleotide changes into the anti-CD40 antibody DNA, or by peptide synthesis. Such variants include, for example, deletions from, and/or insertions into and/or substitutions of, residues within the amino acid sequences of the anti-CD40 antibodies of the examples herein. Any combination of deletions, insertions, and substitutions is made to arrive at the final construct, provided that the final construct possesses the desired characteristics. The amino acid changes also may alter post-translational processes of the humanized or variant anti-CD40 antibody, such as changing the number or position of glycosylation sites.

A useful method for identification of certain residues or regions of the anti-CD40 antibody that are preferred locations for mutagenesis is called "alanine scanning mutagenesis," as described by Cunningham and Wells (Science, 244:1081-1085 (1989)). Here, a residue or group of target residues are identified (e.g., charged residues such as arg, asp, his, lys, and glu) and replaced by a neutral or negatively charged amino acid (typically alanine) to affect the interaction of the amino acids with CD40 antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation per se need not be predetermined. For example, to analyze the performance of a mutation at a given site, alanine scanning or random mutagenesis is conducted at the target codon or region and the expressed anti-CD40 antibody variants are screened for the desired activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an anti-CD40 antibody fused to an epitope tag. Other insertional variants of the anti-CD40 antibody molecule include a fusion to the Nor C-terminus of the anti-CD40 antibody of an enzyme or a polypeptide which increases the serum half-life of the antibody.

Another type of variant is an amino acid substitution variant. These variants have at least one amino acid residue in the anti-CD40 antibody molecule removed and a different residue inserted in its place. The sites of greatest interest for substitutional mutagenesis include the hypervariable regions, but FR alterations are also contemplated. Conservative substitutions are shown in Table 5 under the heading of "preferred substitutions". If such substitutions result in a change in biological activity, then more substantial changes, denominated "exemplary substitutions", or as further described below in reference to amino acid classes, may be introduced and the products screened.

**TABLE 5:**

| Original Residue | Exemplary Substitutions | Preferred Substitutions |
|---|---|---|
| Ala (A) | val; leu; ile | **val** |
| Arg (R) | lys; gin; asn | **lys** |
| Asn (N) | gin; his; asp, lys; arg | **gin** |
| Asp (D) | glu; asn | **glu** |
| Cys (C) | ser; ala | **ser** |
| Gln (Q) | asn; glu | **asn** |
| Glu (E) | asp; gln | **asp** |
| Gly (G) | ala | **ala** |
| His (H) | arg; asn; gin; lys; | **arg** |
| Ile (I) | leu; val; met; ala; phe; norleucine | **leu** |
| Leu (L) | ile; norleucine; val; met; ala; phe | **ile** |
| Lys (K) | arg; gin; asn | **arg** |
| Met (M) | leu; phe; ile | **leu** |
| Phe (F) | tyr; leu; val; ile; ala; | **tyr** |
| Pro (P) | ala | **ala** |
| Ser (S) | thr | **thr** |
| Thr (T) | ser | **ser** |
| Trp (W) | tyr; phe | **tyr** |
| Tyr (Y) | phe;trp; thr; ser | **phe** |
| Val (V) | leu; ile; met; phe ala; norleucine; | **leu** |

In protein chemistry, it is generally accepted that the biological properties of the antibody can be accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gin, his, lys, arg;
(5) residues that influence chain orientation: gly, pro; and
(6) aromatic: trp, tyr, phe.

Non-conservative substitutions will entail exchanging a member of one of these classes for another class.

Any cysteine residue not involved in maintaining the proper conformation of the humanized or variant anti-CD40 antibody also may be substituted, generally with serine, to improve the oxidative stability of the molecule, prevent aberrant crosslinking, or provide for established points of conjugation to a target compound. Conversely, cysteine bond(s) may be added to the antibody to improve its stability (particularly where the antibody is an antibody fragment such as an Fv fragment).

A type of substitutional variant involves substituting one or more hypervariable region residues of a parent antibody (e.g., a humanized or human antibody). Generally, the resulting variant(s) selected for further development will have improved biological properties relative to the parent antibody from which they are generated. A convenient way for generating such substitutional variants is affinity maturation using phage display. Briefly, several hypervariable region sites (e.g., 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (e.g., binding affinity). In order to identify candidate hypervariable region sites for modification, alanine scanning mutagenesis can be performed to identify hypervariable region residues contributing significantly to antigen binding. Alternatively, or in addition, it may be beneficial to analyze a crystal structure of the antigen-antibody complex to identify contact points between the antibody and human CD40. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antibody alters the original glycosylation pattern of the antibody. By "altering" is meant deleting one or more carbohydrate moieties found in the antibody, and/or adding one or more glycosylation sites that are not present in the antibody.

In some embodiments, it may be desirable to modify the antibodies of the invention to add glycosylations sites. Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. Thus, in order to glycosylate a given protein, e.g., an antibody, the amino acid sequence of the protein is engineered to contain one or more of the above-described tripeptide sequences (for N-linked glycosylation sites). The alteration may also be made by the addition of, or substitution by, one or more serine or threonine residues to the sequence of the original antibody (for O-linked glycosylation sites).

Nucleic acid molecules encoding amino acid sequence variants of the anti-CD40 antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the anti-CD40 antibody.

As used herein, the terms "identical" or "percent identity," in the context of two or more nucleic acids or polypeptide sequences, refer to two or more sequences or subsequences that are the same or have a specified percentage of nucleotides or amino acid residues that are the same, when compared and aligned for maximum correspondence. To determine the percent identity, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity=# of identical positions/total # of positions (e.g., overlapping positions)x100). In some embodiments, the two sequences that are compared are the same length after gaps are introduced within the sequences, as appropriate (e.g., excluding additional sequence extending beyond the sequences being compared). For example, when variable region sequences are compared, the leader and/or constant domain sequences are not considered. For sequence comparisons between two sequences, a "corresponding" CDR refers to a CDR in the same location in both sequences (e.g., CDR-H1 of each sequence).

The determination of percent identity or percent similarity between two sequences can be accomplished using a mathematical algorithm. A preferred, non-limiting example of a mathematical algorithm utilized for the comparison of two sequences is the algorithm of Karlin and Altschul, 1990, Proc. Natl. Acad. Sci. USA 87:2264-2268, modified as in Karlin and Altschul, 1993, Proc. Natl. Acad. Sci. USA 90:5873-5877. Such an algorithm is incorporated into the NBLAST and XBLAST programs of Altschul et al., 1990, J. Mol. Biol. 215:403-410. BLAST nucleotide searches can be performed with the NBLAST program, score=100, wordlength=12, to obtain nucleotide sequences homologous to a nucleic acid encoding a protein of interest. BLAST protein searches can be performed with the XBLAST program, score=50, wordlength=3, to obtain amino acid sequences homologous to protein of interest. To obtain gapped alignments for comparison purposes, Gapped BLAST can be utilized as described in Altschul et al., 1997, Nucleic Acids Res. 25:3389-3402. Alternatively, PSI-Blast can be used to perform an iterated search which detects distant relationships between molecules (Id.). When utilizing BLAST, Gapped BLAST, and PSI-Blast programs, the default parameters of the respective programs (e.g., XBLAST and NBLAST) can be used. Another preferred, non-limiting example of a mathematical algorithm utilized for the comparison of sequences is the algorithm of Myers and Miller, CABIOS (1989). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Additional algorithms for sequence analysis are known in the art and include ADVANCE and ADAM as described in Torellis and Robotti, 1994, Comput. Appl. Biosci. 10:3-5; and FASTA described in Pearson and Lipman, 1988, Proc. Natl. Acad. Sci. USA 85:2444-8. Within FASTA, ktup is a control option that sets the sensitivity and speed of the search. If ktup=2, similar regions in the two sequences being compared are found by looking at pairs of aligned residues; if ktup=1, single aligned amino acids are examined. ktup can be set to 2 or 1 for protein sequences, or from 1 to 6 for DNA sequences. The default if ktup is not specified is 2 for proteins and 6 for DNA. Alternatively, protein sequence alignment may be carried out using the CLUSTAL W algorithm, as described by Higgins et al., 1996, Methods Enzymol. 266:383-402.

### Therapeutic Uses

The humanized anti-CD40 antibody or agent is administered by any suitable means, including parenteral, subcutaneous, intraperitoneal, intrapulmonary, and intranasal, and, if desired for local immunosuppressive treatment, intralesional administration (including perfusing or otherwise contacting the graft with the antibody before transplantation). The humanized anti-CD40 antibody or agent can be administered, for example, as an infusion or as a bolus. Parenteral infusions include intramuscular, intravenous, intraarterial, intraperitoneal, or subcutaneous administration. In addition, the humanized anti-CD40 antibody is suitably administered by pulse infusion, particularly with declining doses of the antibody. In one aspect, the dosing is given by injections, most preferably intravenous or subcutaneous injections, depending in part on whether the administration is brief or chronic.

For the prevention or treatment of disease, the appropriate dosage of antibody will depend on a variety of factors such as the type of disease to be treated, as defined above, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments.

Depending on the type and severity of the disease, about 1 µg/kg to 20 mg/kg (e.g., 0.1-15 mg/kg) of antibody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays. An exemplary dosing regimen is that disclosed in WO 94/04188.

The term "suppression" is used herein in the same context as "amelioration" and "alleviation" to mean a lessening of one or more characteristics of the disease.

The antibody composition will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "therapeutically effective amount" of the antibody to be administered will be governed by such considerations, and is the minimum amount necessary to prevent, ameliorate, or treat the disorder associated with CD40 expression.

The antibody need not be, but is optionally, formulated with one or more agents currently used to prevent or treat lupus nephritis. The effective amount of such other agents depends on the amount of humanized anti-CD40 antibody present in the formulation and other factors discussed above. These are generally used in the same dosages and with administration routes as used hereinbefore or about from 1 to 99% of the heretofore employed dosages.

Treatment or prevention of lupus nephritis according to the methods described herein, is achieved by administering to a subject in need of such treatment or prevention an effective amount of the anti-CD40 antibody or agent, whereby the antibody (i) binds to activated immune cells that express CD40 and that are associated with the disease state and (ii) exerts an immunosuppressive effect on the activated immune cells.

Pharmaceutical Compositions and Administration Thereof

A composition comprising a CD40 binding agent (e.g., an anti-CD40 antibody) can be administered to a subject having or at risk of having lupus nephritis. The disclosure further provides for the use of a CD40 binding agent (e.g., an anti-CD40 antibody) in the manufacture of a medicament for prevention or treatment lupus nephritis. The term "subject" as used herein means any mammalian patient to which a CD40-binding agent can be administered, including, e.g., humans and non-human mammals, such as primates, rodents, and dogs. Subjects specifically intended for treatment using the methods described herein include humans. The antibodies or agents can be administered either alone or in combination with other compositions in the prevention or treatment of lupus nephritis.

Preferred antibodies for use in such pharmaceutical compositions are those that comprise humanized antibody or antibody fragment having the heavy chain variable region amino acid sequence of SEQ ID NO:35.

In specific embodiments, the CD40 binding agent composition is administered by injection, by means of a catheter, by means of a suppository, or by means of an implant, the implant being of a porous, non-porous, or gelatinous material, including a membrane, such as a sialastic membrane, or a fiber. Typically, when administering the composition, materials to which the anti-CD40 antibody or agent does not absorb are used.

In other embodiments, the anti-CD40 antibody or agent is delivered in a controlled release system. In one embodiment, a pump may be used (see, e.g., Langer, 1990, Science 249:1527-1533; Sefton, 1989, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used. (See, e.g., Medical Applications of Controlled Release (Langer and Wise eds., CRC Press, Boca Raton, Fla., 1974); Controlled Drug Bioavailability, Drug Product Design and Performance (Smolen and Ball eds., Wiley, New York, 1984); Ranger and Peppas, 1983, Macromol. Sci. Rev. Macromol. Chem. 23:61. See also Levy et al., 1985, Science 228:190; During et al., 1989, Ann. Neurol. 25:351; Howard et al., 1989, J. Neurosurg. 71:105.) Other controlled release systems are discussed, for example, in Langer, supra.

A CD40 binding agent (e.g., an anti-CD40 antibody) can be administered as pharmaceutical compositions comprising a therapeutically effective amount of the binding agent and one or more pharmaceutically compatible ingredients.

In typical embodiments, the pharmaceutical composition is formulated in accordance with routine procedures as a pharmaceutical composition adapted for intravenous or subcutaneous administration to human beings. Typically, compositions for administration by injection are solutions in sterile isotonic aqueous buffer. Where necessary, the pharmaceutical can also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the pharmaceutical is to be administered by infusion, it can be dispensed with an infusion bottle containing sterile pharmaceutical grade water or saline. Where the pharmaceutical is administered by injection, an ampoule of sterile water for injection or saline can be provided so that the ingredients can be mixed prior to administration.

Further, the pharmaceutical composition can be provided as a pharmaceutical kit comprising (a) a container containing a CD40 binding agent (e.g., an anti-CD40 antibody) in lyophilized form and (b) a second container containing a pharmaceutically acceptable diluent (e.g., sterile water) for injection. The pharmaceutically acceptable diluent can be used for reconstitution or dilution of the lyophilized anti-CD40 antibody or agent. Optionally associated with such container(s) can be a notice in the form prescribed by a governmental agency regulating the manufacture, use or sale of pharmaceuticals or biological products, which notice reflects approval by the agency of manufacture, use or sale for human administration.

In one embodiment, the pharmaceutical composition comprises an aqueous composition having a concentration of anti-CD40 antibody from about 10 mg/ml to about 200 mg/ml; or from about 100 mg/ml to about 200 mg/ml; or from about 120 mg/ml to about 180 mg/ml; or about 120 mg/ml, 130 mg/ml, 140 mg/ml, 150 mg/ml, 160 mg/ml, 170 mg/ml, 180 mg/ml, 190 mg/ml or 200 mg/ml.

In addition to the anti-CD40 antibody, the pharmaceutical composition may further comprise a buffer, a stabilizing agent, and a, optionally, a pH adjusting agent. Nonlimiting examples of buffering agents include one or more salts such as sodium chloride, arginine hydrochloride, sodium thiocyanate, ammonium thiocyanate, ammonium sulfate, ammonium chloride, calcium chloride, zinc chloride and sodium acetate; or the salts of suitable acids such as acetic acid and amino acids. The buffering agent is added in an amount sufficient to provide a viscosity suitable for administering the formulation to the patient, for example, by injection. The pharmaceutical composition may comprise from about 100 mM up to about 200 mM of salt or buffer, or from about 120 mM up to about 180 mM salt or buffer. In one embodiment, the pharmaceutical composition comprises a buffer comprising sodium acetate at a concentration of from about 20 mM to about 30 mM and sodium chloride at a concentration of from about 120 mM to about 140 mM. In another embodiment, the pharmaceutical composition comprises a buffer comprising sodium acetate at a concentration of about 25mM and sodium chloride at a concentration of about 130 mM.

A nonlimiting example of a suitable stabilizing agent is polysorbate 20 (Tween 20). The stabilizing agent is present in an amount sufficient to maintain the chemical and physical stability of the pharmaceutical composition. The pharmaceutical composition may comprise from about 0.001 % to about 0.1 % (w/v) of stabilizing agent; or from about 0.0015 % to about 0.015 % (w/v) of stabilizing agent; or about 0.01 % (w/v) of stabilizing agent.

In one embodiment, the pharmaceutical composition comprises the anti-CD40 antibody in an amount from about 120 mg/ml to about 180 mg/ml; a buffer comprising sodium acetate at a concentration of from about 20 mM to about 30 mM and sodium chloride at a concentration of from about 120 mM to about 140 mM; and a surfactant which is polysorbate 20 at a concentration for from about 0.0015 to about 0.015% (w/v). In another embodiment, the anti-CD40 antibody formulation comprises the anti-CD40 antibody in an amount of about 120 mg/ml, 130 mg/ml, 140 mg/ml, 150 mg/ml, 160 mg/ml, 170 mg/ml, 180 mg/ml, 190 mg/ml or 200 mg/ml; a buffer comprising sodium acetate at a concentration of about 25 mM and sodium chloride at a concentration of about 130 mM; and a surfactant which is polysorbate 20 at a concentration of about 0. 01% (w/v).

In another embodiment, each of the pharmaceutical compositions described above may comprise from about 70 mg to about 250 mg of the anti-CD40 antibody; or from about 80 to 240 mg of the anti-CD40 antibody. In another embodiment, pharmaceutical compositions described above comprise 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, or 250 mg of the anti-CD40 antibody.

Each of the pharmaceutical compositions described above has a pH of from about 4.0 to about 12.0; or from about 5 to about 6.0; or about 5.5. The pH may be adjusted by addition of a sufficient amount of a suitable pH adjusting agent such as an acid (e.g., hydrochloric acid) or base (e.g., sodium hydroxide).

In another embodiment, the invention relates to a method of using any one of the ant-CD40 antibody pharmaceutical compositions described herein for treating or prevention lupus nephritis.

The amount of the CD40 binding agent (e.g., anti-CD40 antibody) that is effective in the treatment or prevention of lupus nephritis can be determined by standard clinical techniques. In addition, in vitro assays may optionally be employed to help identify optimal dosage ranges. The precise dose to be employed in the formulation will also depend on the route of administration, and the stage of lupus nephritis, and should be decided according to the judgment of the practitioner and each patient's circumstances. Effective doses may be extrapolated from dose-response curves derived from in vitro or animal model test systems.

For example, toxicity and therapeutic efficacy of the anti-CD40 antibody or agent can be determined in cell cultures or experimental animals by standard pharmaceutical procedures for determining the ED₅₀ (the dose therapeutically effective in 50% of the population). A CD40-binding agent (e.g., an anti-CD40 antibody) that exhibits a large therapeutic index is preferred. Where a CD40-binding agent exhibits toxic side effects, a delivery system that targets the CD40-binding agent to the site of affected tissue can be used to minimize potential damage non-CD40-expressing cells and, thereby, reduce side effects.

The data obtained from the cell culture assays and animal studies can be used in formulating a range of dosage for use in humans. The dosage of the CD40 binding agent typically lies within a range of circulating concentrations that include the ED₅₀ with little or no toxicity. The dosage may vary within this range depending upon the dosage form employed and the route of administration utilized. For any CD40 binding agent used in the method, the therapeutically effective dose can be estimated initially from cell culture assays. A dose can be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma can be measured, for example, by high performance liquid chromatography, ELISA and the like.

Generally, the dosage of an anti-CD40 antibody or CD40 binding agent administered to a patient with lupus nephritis is typically about 0.1 mg/kg to about 100 mg/kg of the subject's body weight. The dosage administered to a subject is about 0.1 mg/kg to about 50 mg/kg, about 1 mg/kg to about 30 mg/kg, about 1 mg/kg to about 20 mg/kg, about 1 mg/kg to about 15 mg/kg, or about 1 mg/kg to about 10 mg/kg of the subject's body weight.

Exemplary doses include, but are not limited to, from 1 ng/kg to 100 mg/kg. In some embodiments, a dose is about 0.5 mg/kg, about 1 mg/kg, about 2 mg/kg, about 3 mg/kg, about 4 mg/kg, about 5 mg/kg, about 6 mg/kg, about 7 mg/kg, about 8 mg/kg, about 9 mg/kg, about 10 mg/kg, about 11 mg/kg, about 12 mg/kg, about 13 mg/kg, about 14 mg/kg, about 15 mg/kg or about 16 mg/kg. The dose can be administered, for example, daily, once per week (weekly), twice per week, thrice per week, four times per week, five times per week, six times per week, biweekly or monthly, every two months, or every three months. In specific embodiments, the dose is about 0.5 mg/kg/week, about 1 mg/kg/week, about 2 mg/kg/week, about 3 mg/kg/week, about 4 mg/kg/week, about 5 mg/kg/week, about 6 mg/kg/week, about 7 mg/kg/week, about 8 mg/kg/week, about 9 mg/kg/week, about 10 mg/kg/week, about 11 mg/kg/week, about 12 mg/kg/week, about 13 mg/kg/week, about 14 mg/kg/week, about 15 mg/kg/week or about 16 mg/kg/week. In some embodiments, the dose ranges from about 1 mg/kg/week to about 15 mg/kg/week.

In another embodiment, the dose is from about 70 mg to about 250 mg per week; or from about 80 to 240 mg per week. In another embodiment, the dose is about 80 mg per week, 120 mg per week, 130 mg per week, 140 mg per week, 160 mg per week, 170 mg per week, 180 mg per week, 200 mg per week, 210 mg per week, 220 mg per week, mg per week, 240 mg per week, or 250 mg per week.

In some embodiments, the pharmaceutical compositions comprising the CD40 binding agent can further comprise a therapeutic agent, either conjugated or unconjugated to the binding agent. The anti-CD40 antibody or CD40 binding agent can be co-administered in combination with one or more therapeutic agents for the treatment or prevention of lupus nephritis.

Such combination therapy administration can have an additive or synergistic effect on disease parameters (e.g., severity of a symptom, the number of symptoms, or frequency of relapse).

With respect to therapeutic regimens for combinatorial administration, in a specific embodiment, an anti-CD40 antibody or CD40 binding agent is administered concurrently with a therapeutic agent. In another specific embodiment, the therapeutic agent is administered prior or subsequent to administration of the anti-CD40 antibody or CD40 binding agent, by at least an hour and up to several months, for example at least an hour, five hours, 12 hours, a day, a week, a month, or three months, prior or subsequent to administration of the anti-CD40 antibody or CD40 binding agent.

In some embodiments, the additional therapeutic agent is an immunosuppressive agent. The immunosuppressive agent can be, for example, gancyclovir, etanercept, tacrolimus, cyclosporine, rapamycin, mycophenolate (MMF), cyclophosphamide (CyP), azathioprine, hydroxychloroquine, mizoribine, mycophenolate mofetil or methotrexate. Alternatively, the immunosuppressive agent can be, for example, a glucocorticoid (e.g., cortisol or aldosterone) or a glucocorticoid analogue (e.g., prednisone or dexamethasone). In another embodiment the immunosuppresive agent can be an angiotensin-converting enzyme (ACE) inhibitor (e.g., captopril, quinapril or enalapril) or an angiotensin II receptor blocker (ARB) (e.g., losartan or candesartan)

In another embodiment, the additional therapeutic agent selected from the group consisting of mycophenolate (MMF), cyclophosphamide (CyP), a glucocorticoid (GC), and corticosteroids, or any combination thereof.

In one embodiment, the additional therapeutic agent is mycophenolate (MMF).

In another embodiment, the additional therapeutic agent is cyclophosphamide (CyP).

In another embodiment, the additional therapeutic agent is a glucocorticoid (GC).

In another embodiment, the additional therapeutic agent is a corticosteroid.

### Articles of Manufacture

In another aspect, an article of manufacture containing materials useful for the treatment of the disorders described above is included. The article of manufacture comprises a container and a label. Suitable containers include, for example, bottles, vials, syringes, and test tubes. The containers may be formed from a variety of materials such as glass or plastic. The container holds a composition that is effective for treating the condition and may have a sterile access port. For example, the container may be an intravenous solution bag or a vial having a stopper pierceable by a hypodermic injection needle. The active agent in the composition is the humanized anti-CD40 antibody. The label on or associated with the container indicates that the composition is used for treating the condition of choice. The article of manufacture may further comprise a second container comprising a pharmaceutically-acceptable buffer, such as phosphate-buffered saline, Ringer's solution, and dextrose solution. It may further include other materials desirable from a commercial and user standpoint, including other buffers, diluents, filters, needles, syringes, and package inserts with instructions for use.

The invention is further described in the following examples, which are not intended to limit the scope of the invention.

### EXAMPLES

### Example 1: Production of Humanized Anti-CD40 Antibody

The humanized anti-CD4 antibodies of the invention cab be prepared according to the procedures described in US20110243932. Antibody A, Antibody B and Antibody C were humanized antibodies derived from mouse antibody 20E2 (Antibody A and Antibody B) or 2H11 (Antibody C) cloned into a human IgG1-KO (KO=knock-out)/kappa backbone. IgG1-KO has two mutations in the Fc region, Leu234Ala and Leu235Ala to reduce FcyR and complement binding.

The results of such humanization resulted in various humanized heavy and light chain variable sequences shown below:
SEQ ID NO: 41 (variable light chain sequence): 
DIVMTQSPDSLAVSLGERVTMSCKSSQSLLNSGNQKNYLTWHQQKPGQPPKLLIYWTSTRESGVPDRFSGSGSGTDF
TLTISSLQAEDVAVYYCQNDYTYPLTFGGGTKVEIK
SEQ ID NO: 42 (variable heavy chain sequence): 
EVQLVKSGGGLVKPGGSLRLSCAASGFTFSDYGMHWVRQAPGKGLEWVAYISSGNRIIYYADTVKGRFTISRDNAKN
SLYLQMNSLRAEDTALYYCARQDGYRYAMDYWGQGTLVTVSS
SEQ ID NO:43 (variable light chain sequence) 
DIVMTQSPDSLAVSLGERATMSCKSSQSLLNSGNQKNYLTWHQQKPGQPPKLLIYWTSTRESGVPDRFSGSGSGTDF
TLTISSLQAEDVAVYYCQNDYTYPLTFGGGTKVEIK
SEQ ID NO: 44 (variable heavy chain sequence) 
EVQLVESGGGLVKPGGSLRLSCAASGFTFSDYGMHWVRQAPGKGLEWVAYISSGNRIIYYADTVKGRFTISRDNAKN
SLYLQMNSLRAEDTALYYCARQDGYRYAMDYWAQGTLVTVSS
SEQ ID NO: 45 (variable light chain sequence) 
DIVMTQSPDSLAVSLGEKVTMNCKSSQSLLNSGNQKNYLTWHQQKPGQPPKLLIYWTSTRESGVPDRFSGSGSGTDF
TLTISSLQAEDVAVYYCQNDYTYPLTFGAGTKVEIK
SEQ ID NO: 46 (variable heavy chain sequence) 
EVQLVESGGGLVKPGGSRRLSCAASGFTFSDYGMHWVRQAPGKGLEWVAYISSGNRIIYYADTVKGRFTISRDNAKN

SLYLQMNSLRAEDTALYYCARQDGYRYAMDYWGQGTLVTVSS.
SEQ ID NO: 47 (variable light chain sequence) 
DIVMTQSPDSLAVSLGERVTMNCKSSQSLLNSGNQKNYLTWHQQKPGQPPKLLIYWTSTRESGVPDRFSGSGSGTDF
TLTISSLQAEDVAVYYCQNDYTYPLTFGGGTKVEIK
SEQ ID NO: 48 (variable heavy chain sequence) 
EVQLVESGGGLVKPGGSLRLSCAASGFTFSDYGMHWVRQAPGKGLEWVAYISSGNRIIYYADTVKGRFTISRDNAKN
SLYLQMNSLRAEDTALYYCARQDGYRYAMDYWGQGTLVTVSS
SEQ ID NO: 49 (variable light chain sequence) 
DIVMTQSPDSLAVSLGERVTMNCKSSQSLLNSGNQKNYLTWHQQKPGQPPKLLIYWTSTRESGVPDRFSGSGSGTDF
TLTISSLQAEDVAVYYCQNDYTYPLTFGAGTKVEIK
SEQ ID NO: 50 (variable light chain sequence) 
EVQLVESGGGLVKPGGSRRLSCAASGFTFSDYGMHWVRQAPGKGLEWVAYISSGNRIIYYADTVKGRFTISRDNAKN
SLYLQMNSLRAEDTAVYYCARQDGYRYAMDYWGQGTLVTVSS
SEQ ID NO: 51 (variable light chain sequence) 
DIVMTQSPDSLAVSLGEKVTMNCKSSQSLLNSGNQKNYLTWHQQKPGQPPKLLIYWTSTRESGVPDRFSGSGSGTDF

TLTISSLQAEDLAVYYCQNDYTYPLTFGAGTKVEIK.
SEQ ID NO: 52 (variable light chain sequence) 
DIVMTQSPDSLAVSLGEKVTINCKSSQSLLNSGNQKNYLTWHQQKPGQPPKLLIYWTSTRESGVPDRFSGSGSGTDF
TLTISSLQAEDVAVYYCQNDYTYPLTFGGGTKVEIK
SEQ ID NO: 53 (variable heavy chain sequence) 
EVQLVESGGGLVKPGGSLRLSCAASGFTFSDYGMHWVRQAPGKGLEWVAYISSGNRIIYYADTVKGRFTISRDNAKN
SLYLQMNSLRAEDTAVYYCARQDGYRYAMDYWGQGTLVTVSS
SEQ ID NO: 54 (variable light chain sequence) 
QIQMTQSPSSLSASVGDRVTITCSASSSVSYMLWFQQKPGKAPKLWIYSTSNLASGVPARFSGSGSGTDFTLTISSL
QPEDFATYYCQQRTFYPYTFGGGTKVEIK
SEQ ID NO:55 (variable light chain sequence) 
DIQMTQSPSSLSASVGDRVTITCSASSSVSYMLWFQQKPGKAPKLLIYSTSNLASGVPARFSGSGSGTDFTLTISSL
QPEDFATYYCQQRTFYPYTFGGGTKVEIK
SEQ ID NO:56 (variable light chain sequence) 
DIQMTQSPSSLSASVGDRVTITCSASSSVSYMLWFQQKPGKAPKLLIYSTSNLASGVPSRFSGSGSGTDFTLTISSL
QPEDFATYYCQQRTFYPYTFGGGTKVEIK
SEQ ID NO:57 (variable heavy chain sequence) 
QVQLVQSGAEVKKPGASVKVSCTASGFNITDYYVHWVKQRPGQGLEWMGRIDPEDGDSKYAPKFQGKATMTADTSTS
TVYMELSSLRSEDTAVYYCTTSYYVGTYGYWGQGTLVTVSS.
SEQ ID NO:58 (variable heavy chain sequence) 
QVQLVQSGAEVKKPGASVKVSCTASGFNIKDYYVHWVKQAPGQGLEWMGRIDPEDGDSKYAPKFQGKATMTADTSTS
TVYMELSSLRSEDTAVYYCTTSYYVGTYGYWGQGTLVTVSS
SEQ ID NO:59 (variable heavy chain sequence) 
QVQLVQSGAEVKKPGASVKVSCTASGFNITDYYVHWVKQRPGQGLEWMGRIDPEDGDSKYAPKFQGKVTMTADTSTS
TVYMELSSLRSEDTAVYYCTTSYYVGTYGYWGQGTLVTVSS.
SEQ ID NO:60 (variable heavy found sequence) 
QVQLVQSGAEVKKPGASVKVSCTASGFNIKDYYVHWVKQAPGQGLEWIGRIDPEDGDSKYAPKFQGKATMTADTSTS
TVYMELSSLRSEDTAVYYCTTSYYVGTYGYWGQGTLVTVSS
SEQ ID NO:61 (variable heavy sequence) 
QVQLVQSGAEVKKPGASVKVSCTASGFNITDYYVHWVKQAPGQGLEWMGRIDPEDGDSKYAPKFQGKATMTADTSTS
TVYMELSSLRSEDTAVYYCTTSYYVGTYGYWGQGTLVTVSS
SEQ ID NO:62 (variable heavy sequence): 
QVQLVQSGAEVKKPGASVKVSCTASGFNITDYYVHWVKQRPGQGLEWMGRIDPEDGDTKFAPKFQGKATMTADTSTS
TVYMELSSLRSEDTAVYYCTTSYYVGTYGYWGQGTLVTVSS
SEQ ID NO:63 (variable heavy sequence) 
QVQLVQSGAEVKKPGASVKVSCTASGFNITDYYVHWVKQRPGQGLEWMGRIDPEDGDTKFAPKFQGKVTMTADTSTS
TVYMELSSLRSEDTAVYYCTTSYYVGTYGYWGQGTLVTVSS
SEQ ID NO:64 (variable heavy sequence) 
QVQLVQSGAEVKKPGASVKVSCTASGFNIKDYYVHWVKQAPGQGLEWIGRIDPEDGDTKFAPKFQGKATMTADTSTS
TVYMELSSLRSEDTAVYYCTTSYYVGTYGYWGQGTLVTVSS
SEQ ID NO:65 (variable heavy sequence) 
QVQLVQSGAEVKKPGASVKVSCTASGFNIKDYYVHWVKQAPGQGLEWMGRIDPEDGDTKFAPKFQGKATMTADTSTS
TVYMELSSLRSEDTAVYYCTTSYYVGTYGYWGQGTLVTVSS
SEQ ID NO:66 (variable heavy sequence) 
QVQLVQSGAEVKKPGASVKVSCTASGFNITDYYVHWVKQAPGQGLEWMGRIDPEDGDTKFAPKFQGKATMTADTSTS
TVYMELSSLRSEDTAVYYCTTSYYVGTYGYWGQGTLVTVSS
SEQ ID NO:67 (variable heavy sequence) 
EVQLVQSGAEVKKPGATVKISCKVSGFNIKDYYIHWVKQRPGKGLEWMGRIDPEDGDTKYDPKFQGRVTMTADTSTD
TAYMELSSLRSEDTAVYYCTTSYYVGTYGYWGQGTTVTVSS
SEQ ID NO:68 (variable heavy sequence) 
EVQLVQSGAEVKKPGATVKISCTVSGFNIKDYYIHWVKQRPGKGLEWMGRIDPEDGDTKYDPKFQGRVTMTADTSTD
TAYMELSSLRSEDTAVYYCTTSYYVGTYGYWGQGTTVTVSS
SEQ ID NO:69 (variable heavy sequence) 
EVQLVQSGAEVKKPGATVKISCTVSGFNIKDYYIHWVKQRPGKGLEWMGRIDPEDGDTKYDPKFQGKVTMTADTSTD
TAYMELSSLRSEDTAVYYCTTSYYVGTYGYWGQGTTVTVSS
SEQ ID NO:70 (variable heavy sequence) 
EVQLVQSGAEVKKPGATVKISCTVSGFNIKDYYIHWVKQAPGKGLEWMGRIDPEDGDTKYDPKFQGKATMTADTSTD
TAYMELSSLRSEDTAVYYCTTSYYVGTYGYWGQGTTVTVSS
SEQ ID NO:71 (variable heavy sequence) 
EVQLVQSGAEVKKPGATVKISCTVSGFNIKDYYIHWVKQRPGKGLEWMGRIDPEDGDTKYDPKFQGKATMTADTSTD

TAYMELSSLRSEDTAVYYCTTSYYVGTYGYWGQGTTVTVSS
SEQ ID NO:72 (variable heavy sequence) 
EVQLVQSGAEVKKPGATVKISCTVSGFNIKDYYIHWVKQAPGKGLEWIGRIDPEDGDTKYDPKFQGKATMTADTSTD
TAYMELSSLRSEDTAVYYCTTSYYVGTYGYWGQGTTVTVSS
SEQ ID NO:73 (variable heavy sequence) 
EVQLVQSGAEVKKPGATVKISCKVSGFNIKDYYIHWVQQAPGKGLEWMGRIDPEDGDTKYDPKFQGRVTMTADTSTD
TAYMELSSLRSEDTAVYYCTTSYYVGTYGYWGQGTTVTVSS
SEQ ID NO:74 (variable light sequence) 1 from antibody 10F2Hum: 
DIQMTQSPSSLSASVGDRVTITCSATSSVSYILWFQQKPGKAPKLLIYSTSNLASGVPSRFSGSGSGTDFTLTISSL
QPEDFATYYCQQRTFYPYTFGGGTKVEIK
SEQ ID NO: 75 (variable light sequence) 2 from antibody 10F2Hum: 
DIQMTQSPSSLSASVGDRVTITCSATSSVSYILWFQQKPGKAPKLLIYSTSNLASGVPARFSGSGSGTDFTLTISSL
QPEDFATYYCQQRTFYPYTFGGGTKVEIK
SEQ ID NO: 76 (variable light sequence)

QIQMTQSPSSLSASVGDRVTITCSATSSVSYILWFQQKPGKAPKLWIYSTSNLASGVPARFSGSGSGTDFTLTISSL
QPEDFATYYCQQRTFYPYTFGGGTKVEIK

Exemplary humanized antibodies are those that have the heavy and light chain sequences set forth in the following table. The bold underlined sequences in the following table are the variable domains whereas the normal, non-underlined sequences are the constant domains:

| Identity | Sequence | SEQ ID NO: |
|---|---|---|
| **Antibody A (Light Chain)** | DIVMTQSPDSLAVSLGERATMSCKSSQSLLNSGNQKNYLTW HQQKPGQPPKLLIYWTSTRESGVPDRFSGSGSGTDFTLTIS SLQAEDVAVYYCQNDYTYPLTFGGGTKVEIKRTVAAPSVFI FPPSDEQLKSGTASVVCLLNNFYPREAKVQWKVDNALQSGN SQESVTEQDSKDSTYSLSSTLTLSKADYEKHKVYACEVTHQ GLSSPVTKSFNRGEC | 26 |
| **Antibody A (Heavy Chain, IgG1KO)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTALYYCARQDGYRYAMDYWAQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSREEMTKNQVSLTC LVKGFYPSDIAVEWESNGQPENNYKTTPPVLDSDGSFFLYS KLTVDKSRWQQGNVFSCSVMHEALHNHYTQKSLSLSPGK | 27 |
| **Antibody A (Heavy Chain, IgG1)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTALYYCARQDGYRYAMDYWAQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPELLGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSREEMTKNQVSLTC LVKGFYPSDIAVEWESNGQPENNYKTTPPVLDSDGSFFLYS KLTVDKSRWQQGNVFSCSVMHEALHNHYTQKSLSLSPGK | 28 |
| **Antibody A (Heavy Chain, IgG4DM)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTALYYCARQDGYRYAMDYWAQGTLVTVSSASTK GPSVFPLAPCSRSTSESTAALGCLVKDYFPEPVTVSWNSGA LTSGVHTFPAVLQSSGLYSLSSVVTVPSSSLGTKTYTCNVD HKPSNTKVDKRVESKYGPPCPPCPAPEFEGGPSVFLFPPKP KDTLMISRTPEVTCVVVDVSQEDPEVQFNWYVDGVEVHNAK TKPREEQFNSTYRVVSVLTVLHQDWLNGKEYKCKVSNKGLP | 29 |
| | SSIEKTISKAKGQPREPQVYTLPPSQEEMTKNQVSLTCLVK GFYPSDIAVEWESNGQPENNYKTTPPVLDSDGSFFLYSRLT VDKSRWQEGNVFSCSVMHEALHNHYTQKSLSLSLGK | |
| **Antibody A (Heavy, IgGlKOb)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTALYYCARQDGYRYAMDYWAQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSRDELTKNQVSLTC LVKGFYPSDIAVEWESNGQPENNYKTTPPVLDSDGSFFLYS KLTVDKSRWQQGNVFSCSVMHEALHNHYTQKSLSLSPGK | 30 |
| **Antibody B (Light Chain)** | DIVMTQSPDSLAVSLGEKVTINCKSSQSLLNSGNQKNYL TWHQQKPGQPPKLLIYWTSTRESGVPDRFSGSGSGTDFT LTISSLQAEDVAVYYCQNDYTYPLTFGGGTKVEIKRTVA APSVFIFPPSDEQLKSGTASVVCLLNNFYPREAKVQWKV DNALQSGNSQESVTEQDSKDSTYSLSSTLTLSKADYEKH KVYACEVTHQGLSSPVTKSFNRGEC | 31 |
| **Antibody B (Heavy Chain, IgG1KO)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTAVYYCARQDGYRYAMDYWGQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSREEMTKNQVSLTC LVKGFYPSDIAVEWESNGQPENNYKTTPPVLDSDGSFFLYS KLTVDKSRWQQGNVFSCSVMHEALHNHYTQKSLSLSPGK | 32 |
| **Antibody B (Heavy Chain, IgG1)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTAVYYCARQDGYRYAMDYWGQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPELLGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSREEMTKNQVSLTC LVKGFYPSDIAVEWESNGQPENNYKTTPPVLDSDGSFFLYS KLTVDKSRWQQGNVFSCSVMHEALHNHYTQKSLSLSPGK | 33 |
| **Antibody B (Heavy Chain,IgG4 DM)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTAVYYCARQDGYRYAMDYWGQGTLVTVSSASTK GPSVFPLAPCSRSTSESTAALGCLVKDYFPEPVTVSWNSGA LTSGVHTFPAVLQSSGLYSLSSVVTVPSSSLGTKTYTCNVD HKPSNTKVDKRVESKYGPPCPPCPAPEFEGGPSVFLFPPKP KDTLMISRTPEVTCVVVDVSQEDPEVQFNWYVDGVEVHNAK TKPREEQFNSTYRVVSVLTVLHQDWLNGKEYKCKVSNKGLP SSIEKTISKAKGQPREPQVYTLPPSQEEMTKNQVSLTCLVK | 34 |
| | GFYPSDIAVEWESNGQPENNYKTTPPVLDSDGSFFLYSRLT VDKSRWQEGNVFSCSVMHEALHNHYTQKSLSLSLGK | |
| **Antibody B (Heavy Chain, IgGIKOb)** | EVQLVESGGGLVKPGGSLRLSCAASGFTFSDYGMHWVRQAP GKGLEWVAYISSGNRIIYYADTVKGRFTISRDNAKNSLYLQ MNSLRAEDTAVYYCARQDGYRYAMDYWGQGTLVTVSSASTK GPSVFPLAPSSKSTSGGTAALGCLVKDYFPEPVTVSWNSGA LTSGVHTFPAVLQSSGLYSLSSVVTVPSSSLGTQTYICNVN HKPSNTKVDKRVEPKSCDKTHTCPPCPAPEAAGGPSVFLFP PKPKDTLMISRTPEVTCVVVDVSHEDPEVKFNWYVDGVEVH NAKTKPREEQYNSTYRVVSVLTVLHQDWLNGKEYKCKVSNK ALPAPIEKTISKAKGQPREPQVYTLPPSRDELTKNQVSLTC LVKGFYPSDIAVEWESNGQPENNYKTTPPVLDSDGSFFLYS KLTVDKSRWQQGNVFSCSVMHEALHNHYTQKSLSLSPGK | 35 |
| **Antibody C (Light Chain)** | DIQMTQSPSSLSASVGDRVTITCSASSSVSYMLWFQ QKPGKAPKLLIYSTSNLASGVPSRFSGSGSGTDFTL TISSLQPEDFATYYCQQRTFYPYTFGGGTKVEIKRT VAAPSVFIFPPSDEQLKSGTASVVCLLNNFYPREAK VQWKVDNALQSGNSQESVTEQDSKDSTYSLSSTLTL SKADYEKHKVYACEVTHQGLSSPVTKSFNRGEC | 36 |
| **Antibody C (Heavy Chain, IgG1KO)** | QVQLVQSGAEVKKPGASVKVSCTASGFNIKDYYVHWVKQAP GQGLEWMGRIDPEDGDSKYAPKFQGKATMTADTSTSTVYME LSSLRSEDTAVYYCTTSYYVGTYGYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKRVEPKSCDKTHTCPPCPAPEAAGGPSVFLFPP KPKDTLMISRTPEVTCVVVDVSHEDPEVKFNWYVDGVEVHN AKTKPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYSK LTVDKSRWQQGNVFSCSVMHEALHNHYTQKSLSLSPGK | 37 |
| **Antibody C (Heavy Chain, IgG1)** | QVQLVQSGAEVKKPGASVKVSCTASGFNIKDYYVHWVKQAP GQGLEWMGRIDPEDGDSKYAPKFQGKATMTADTSTSTVYME LSSLRSEDTAVYYCTTSYYVGTYGYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKRVEPKSCDKTHTCPPCPAPELLGGPSVFLFPP KPKDTLMISRTPEVTCVVVDVSHEDPEVKFNWYVDGVEVHN AKTKPREEQYNSTYRVVSVLTVLHQDWLNGKEYKCKVSNKA LPAPIEKTISKAKGQPREPQVYTLPPSREEMTKNQVSLTCL VKGFYPSDIAVEWESNGQPENNYKTTPPVLDSDGSFFLYSK LTVDKSRWQQGNVFSCSVMHEALHNHYTQKSLSLSPGK | 38 |
| **Antibody C (Heavy Chain,IgG4 DM)** | QVQLVQSGAEVKKPGASVKVSCTASGFNIKDYYVHWVKQAP GQGLEWMGRIDPEDGDSKYAPKFQGKATMTADTSTSTVYME LSSLRSEDTAVYYCTTSYYVGTYGYWGQGTLVTVSSASTKG PSVFPLAPCSRSTSESTAALGCLVKDYFPEPVTVSWNSGAL TSGVHTFPAVLQSSGLYSLSSVVTVPSSSLGTKTYTCNVDH KPSNTKVDKRVESKYGPPCPPCPAPEFEGGPSVFLFPPKPK DTLMISRTPEVTCVVVDVSQEDPEVQFNWYVDGVEVHNAKT KPREEQFNSTYRVVSVLTVLHQDWLNGKEYKCKVSNKGLPS SIEKTISKAKGQPREPQVYTLPPSQEEMTKNQVSLTCLVKG FYPSDIAVEWESNGQPENNYKTTPPVLDSDGSFFLYSRLTV | 39 |
| | DKSRWQEGNVFSCSVMHEALHNHYTQKSLSLSLGK | |
| **Antibody C (Heavy Chain, IgGIKOb)** | QVQLVQSGAEVKKPGASVKVSCTASGFNIKDYYVHWVKQAP GQGLEWMGRIDPEDGDSKYAPKFQGKATMTADTSTSTVYME LSSLRSEDTAVYYCTTSYYVGTYGYWGQGTLVTVSSASTKG PSVFPLAPSSKSTSGGTAALGCLVKDYFPEPVTVSWNSGAL TSGVHTFPAVLQSSGLYSLSSVVTVPSSSLGTQTYICNVNH KPSNTKVDKRVEPKSCDKTHTCPPCPAPEAAGGPSVFLFPP KPKDTLMISRTPEVTCVVVDVSHEDPEVKFNWYVDGVEVHN AKTKPREEQYNSTYRVVSVLTVLHQDWLNGKEYKCKVSNKA LPAPIEKTISKAKGQPREPQVYTLPPSRDELTKNQVSLTCL VKGFYPSDIAVEWESNGQPENNYKTTPPVLDSDGSFFLYSK LTVDKSRWQQGNVFSCSVMHEALHNHYTQKSLSLSPGK | 40 |

The variable regions were subcloned into one or two different suitable IgG expression vectors:
A) a human IgG1-KO (knock-out)/kappa format with a Leu234Ala, Leu235Ala double mutation in the Fc region to reduce effector function such as FcyR and complement binding
B) a human IgG4-DM (double mutant)/kappa format with a Ser228Pro mutation in the hinge region to reduce the occurrence of IgG4 half-molecules and a Leu235Glu mutation to further reduce FcyR binding

The Antibody A and Antibody B were purified and evaluated by the following criteria:
- Appearance of CCF (turbidity)
- Filtration properties of CCF
- Yield on rProteinA
- Turbidity upon elution and neutralization
- Soluble aggregates (SEC)
- Purity / contamination pattern (SDS)
- Charge pattern (IEF)

### Example 2:

SLE is a systemic autoimmune disease characterized by loss of B cell tolerance to various auto- antigens, particularly nucleic acids and their binding proteins. These auto-antibodies form immune complexes that deposit in various tissues throughout the body and in part with other factors, drive recruitment of inflammatory cells and mediators in the kidney that results in lupus nephritis. The generation of the auto-antibodies is dependent on T cell-B cell interactions within the lymph node and requires CD40-CD40L to drive B cell proliferation and formation of germinal centers. Within the germinal center, CD40-CD40L interactions mediate B cell maturation through Ig isotype switching, somatic mutation, clonal expansion of high-affinity B cells, and terminal differentiation to plasma cells (Tarlington, 1998). Aberrant expression of CD40L on T cells and platelets in lupus patients (Koshy et al., 1996 and Manea et al., 2009) may also serve as a mechanism to drive autoreactive B cells. Finally, increased expression of CD40L in SLE B cells results in spontaneous auto-antibody production in a T cell independent manner (Grammer et al., 2003). See also US20110243932.

Further evidence implicating the CD40 pathway in lupus nephritis is through increased levels and expression of CD40 and CD40L in diseased patients. Along with increased membrane bound CD40L, soluble CD40L is increased in circulation of patients with SLE (Goules et al., 2006) and this increase correlates with disease activity. Increased CD40 expression on monocytes also correlates with disease activity in lupus nephritis (Kuroiwa et al., 2003). In addition to the role of the CD40 pathway in generation of auto-antibodies and inflammation in the kidney, data implicates a role for this pathway in the modulation of non-immune cells which drive inflammation in lupus nephritis. For example, activation of endothelial cells via CD40-CD154 interaction triggers proinflammatory cytokine and chemokine production, matrix metalloproteinase and tissue factor expression and increase in the density of leukocyte adhesion molecules CD62E, CD106, and CD54 (Pulivenet et al, 2008). These events play an important role in the promotion of extravasation and accumulation of activated T cells at the sites of inflammation. Human kidney mesangial cells express low levels of CD40 and upregulate this receptor in response to IFN-γ treatment and activated CD154+ platelets from SLE patients. (Delams et al., 2005). Likewise, in proximal tubular epithelial cells (PTEC), CD40L mediated induction of cytokines such as IL-6, IL-8 is observed.

The effects of CD40 ligation on human endothelial cells (HUVEC) and human proximal tubular epithelial cells (PTEC) can be evaluated by stimulating the cells with CD40L. HUVECs were purchased from Lonza Allendale, NJ HUVEC P901 (Cat. C2519A, lot 0000220212). Cells were cultured in HUVEC medium (Lonza EBM-2 medium with singlebullet supplements) and sub-culture reagents: (Lonza Allendale, NJ Cat. CC-3156, lot 0000243756). Cells were grown in BD Biocoat Collagen I T75 flasks (BD Biosciences San Jose, CA Cat. 356485, lot 1108951). Cells were sub-cultured using a Lonza sub-culture kit as per manufacturer's instructions.

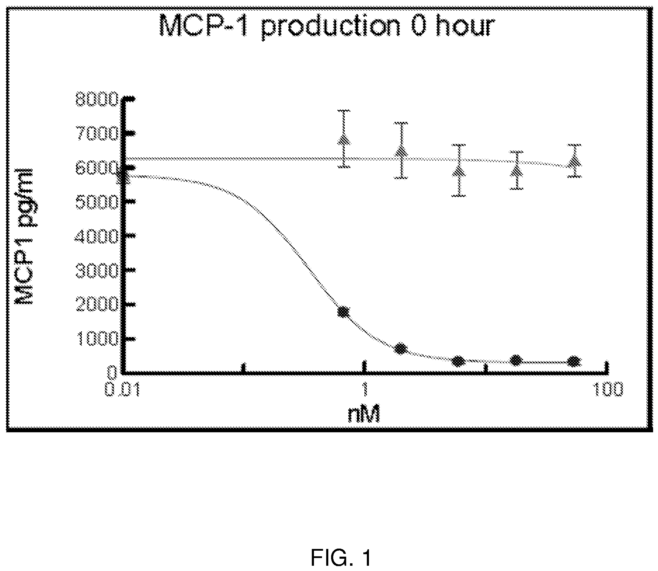

Fig. 1 shows that soluble CD40L induces inflammatory cytokines such as MCP-1 in HUVEC that is effectively blocked by exemplary Antibody B (●) or isotype control (lgG1 human antibody) (▲) in a dose dependent manner.

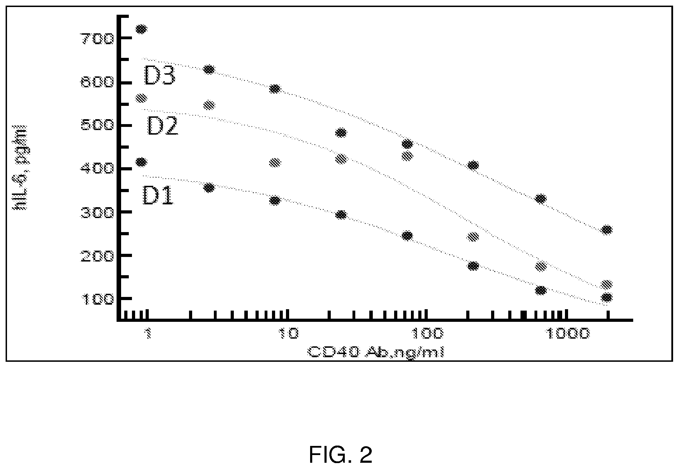

Fig. 2 shows the response to sCD40L in PTECs from the three different human donors. PTECS were purchased from Lonza, cat.#CC-2553, and included three different donors (D1, D2, D3). Cells were grown in a Renal Cell Growth Medium (REGM) media containing 50 ng/ml of sCD40L (Recombinant human mega CD40L, Alexis Biochemical-Enzo Life Science Farmingdale, NY Cat. 522-110-C010, lot 26945, 10 ug/ml). The REGM SingleQuot kit (Lonza, cat.# CC-4127, lot #0000193027) provided a medium comprising 0.5% fetal bovine serum (FBS), hydrocortisone, human epidermal growth factor (hEGF), epinephrine, insulin, triiodothyronine, transferin, gentamicin/amphotericin-B. The results in Fig. 2 shows that the PTECs from the three different human donors produce IL-6 in response to sCD40L stimulation and this response can be inhibited dose-dependently by Antibody B (concentrations of Antibody A from 0 ng/ml to 2000 ng/ml.)

Applicant believes that the results and other disclosures described herein establish a strong rationale for the CD40 pathway playing a central role in the pathogenesis of lupus nephritis.

## Claims

1. A humanized antibody that specifically binds human CD40 for use in treating or preventing lupus nephritis, wherein said antibody comprises a heavy chain variable region of SEQ ID NO: 53 and a light chain variable region of SEQ ID NO: 52.

2. The humanized monoclonal antibody for use according to claim 1, wherein the antibody is an IgG1 antibody.

3. The humanized monoclonal antibody for use according to claim 2, wherein the antibody has a Leu234Ala and Leu235Ala mutation in the Fc region and comprises a kappa light chain.

4. The humanized monoclonal antibody for use according to any one of claims 1 to 3, comprising a heavy chain of SEQ ID NO:35 and a light chain of SEQ ID NO:31.

5. The humanized antibody for use according to any one of claims 1 to 4, wherein said antibody is to be administered by a parenteral route, intravenous route or subcutaneous route of administration.

## Patentansprüche

1. Ein humanisierter Antikörper welcher speziell menschliches CD40 bindet zur Verwendung bei der Behandlung oder der Vorbeugung von Lupus-Nephritis, wobei besagter Antikörper eine variable Region der schweren Kette der SEQ ID NO: 53 und eine variable Region der leichten Kette der SEQ ID NO: 52 umfasst.

2. Der humanisierte monoklonale Antikörper zur Verwendung nach Anspruch 1, wobei der Antikörper ein IgG1 Antikörper ist.

3. Der humanisierte monoklonale Antikörper zur Verwendung nach Anspruch 2, wobei der Antikörper eine Leu234Ala und Leu235Ala Mutation in der Fc-Region besitzt und eine leichte Kappa-Kette aufweist.

4. Der humanisierte monoklonale Antikörper zur Verwendung nach einem der Ansprüche 1 bis 3, aufweisend eine schwere Kette der SEQ ID NO:35 und eine leichte Kette der SEQ ID NO: 31.

5. Der humanisierte Antikörper zur Verwendung nach einem der Ansprüche 1 bis 4, wobei besagter Antikörper zu verabreichen ist auf parenteralem Weg, intravenösem Weg oder subkutanem Weg der Verabreichung.

## Revendications

1. Anticorps humanisé qui se lie spécifiquement au CD40 humain pour une utilisation dans le traitement ou la prévention de la néphrite lupique, dans lequel ledit anticorps comprend une région variable de chaîne lourde de SEQ ID NO : 53 et une région variable de chaîne légère de SEQ ID NO : 52.

2. Anticorps monoclonal humanisé pour son utilisation selon la revendication 1, dans lequel l'anticorps est un anticorps IgG1.

3. Anticorps monoclonal humanisé pour son utilisation selon la revendication 2, dans lequel l'anticorps a une mutation Leu234Ala et Leu235Ala dans la région Fc et comprend une chaîne légère kappa.

4. Anticorps monoclonal humanisé pour son utilisation selon l'une quelconque des revendications 1 à 3, comprenant une chaîne lourde de SEQ ID NO : 35 et une chaîne légère de SEQ ID NO : 31.

5. Anticorps humanisé pour son utilisation selon l'une quelconque des revendications 1 à 4, dans lequel ledit anticorps est destiné à être administré par une voie parentérale, une voie intraveineuse ou une voie sous-cutanée d'administration.
